(19) Europäisches Patentamt
European Patent Office
Office européen des brevets

(11) EP 2 797 577 B1

(12) # EUROPÄISCHE PATENTSCHRIFT

(45) Veröffentlichungstag und Bekanntmachung des Hinweises auf die Patenterteilung:
**13.06.2018 Patentblatt 2018/24**

(21) Anmeldenummer: **12784596.4**

(22) Anmeldetag: **13.11.2012**

(51) Int Cl.:
***A61Q 5/10*** (2006.01) ***A61K 8/86*** (2006.01)
***A61K 8/893*** (2006.01)

(86) Internationale Anmeldenummer:
**PCT/EP2012/072457**

(87) Internationale Veröffentlichungsnummer:
**WO 2013/092005 (27.06.2013 Gazette 2013/26)**

(54) **ZUSAMMENSETZUNGEN ZUR FÄRBUNG KERATINHALTIGER FASERN**
COMPOSITIONS FOR DYEING KERATIN FIBERS
COMPOSITIONS POUR LA COLORATION DE FIBRES KÉRATINIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.12.2011 DE 102011089063**

(43) Veröffentlichungstag der Anmeldung:
**05.11.2014 Patentblatt 2014/45**

(73) Patentinhaber: **Henkel AG & Co. KGaA**
**40589 Düsseldorf (DE)**

(72) Erfinder: **SCHWEINSBERG, Matthias**
**22769 Hamburg (DE)**

(56) Entgegenhaltungen:
**EP-A2- 2 168 633 WO-A2-2011/015514**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft kosmetische Mittel zur Färbung keratinhaltiger Fasern.

Zur Bereitstellung kosmetischer Mittel zur Färbung, insbesondere für keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt. Als Kupplerkomponenten werden in der Regel m-Phenylendiaminderivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte dierektziehende Farbstoffe enthalten. Hierbei handelt es sich um Farbstoffmoleküle, die direkt auf das Substrat aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Eine Verbesserung beispielsweise der Farbintensität oder der Färbekraft der eingesetzten Farbstoffe hat z.B. zur Folge, dass teure Farbstoffe wirtschaftlicher eingesetzt werden können. Die erzielten Färbungen sollen weiterhin ein hohes Maß an Farbechtheit z.B. gegen Schweiß, Waschen, Licht oder Reibung aufweisen und müssen insbesondere im Rahmen der Haarpflege kompatibel mit der Anwendung anderer Haarbehandlungsmitteln sein. Eine gleichmäßige Färbung entlang der keratinhaltigen Fasern stellt ebenso eine Anforderung an ein kommerziell erfolgreiches Färbemittel für keratinhaltige Fasern dar. Keratinische Fasern, insbesondere menschliche Haare, sind als gewachsene Naturprodukte ungleichmäßig bezüglich ihrer strukturellen Beschaffenheit entlang der Faser. Das keratinhaltige Material der Faser in den Haarlängen und den Haarspitzen wurde beispielsweise über einen längeren Zeitraum der Umwelteinflüsse ausgesetzt als die Regionen der Faser in der Nähe der Haarwurzel und weisen daher stärkere Veränderungen der ursprünglich gewachsenen Faserstruktur auf. Unterschiede in der Faserstruktur führen oftmals zu einer ungleichmäßigen Farbaufnahme sowie zu einer ungleichmäßigen Abnutzung der Färbung durch Umwelteinflüsse. Die Färbung wird visuell als ungleichmäßig wahrgenommen.

Aufgabe der vorliegenden Erfindung war es daher, eine die keratinhaltigen Fasern färbende, kosmetische Zusammensetzung bereitzustellen, die eine verbesserte Färbung bewirkt und die vorgenannten Nachteile nicht aufweist.

In der Druckschrift WO-A1-2009/024450 werden sternförmige Polyether-haltige Verbindungen beschrieben, die in Mitteln zur Reinigung von Oberflächen Anwendung finden und die Wiederanschmutzung des gereinigten Substrats eindämmen.

In den Druckschriften WO-A1-2011/015512, WO-A1-2011/015513 und WO-A1-2011/015514 werden alkoxysilylierte, Polyether-haltige Verbindungen zur Anwendung auf dem Haar vorgeschlagen.

Die EP 2 168 633 A2 offenbart waschbeständige Haarfärbeprozesse aufbauend auf Aktivstoffen mit hydrolysierbaren Silylgruppen.

Die Verbesserung der Farbechtheit künstlicher Färbungen auf keratinhaltigen Fasern durch alkoxysilylierte, Polyether-haltige Verbindungen ist aus dem Stand der Technik nicht bekannt.

Ein erster Gegenstand ist daher die Verwendung von Sternpolymeren, umfassend ein als Zentrum fungierendes Strukturfragment und mindestens drei radial an dieses Zentrum gebundene Gruppen, wobei diese Gruppen

(i) jeweils mindestens eine Polyetherstruktureinheit aufweisen, die eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) jeweils mindestens einen Rest aufweisen, enthaltend

- mindestens eine Gruppe *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht, und/oder
- mindestens eine anionische Gruppe und/oder
- mindestens ein quaternisiertes Stickstoffatom,

zur Verbesserung der Farbechtheit künstlicher Färbungen farbgebender Verbindungen auf keratinhaltigen Fasern, insbesondere menschlichen Haaren,
wobei die farbgebenden Verbindungen aus mindestens einem Oxidationsfarbstoffvorprodukt vom Typ der Entwicklerkomponenten und gegebenenfalls zusätzlich mindestens einer Kupplerkomponente ausgewählt werden.

Es stellte sich heraus, dass der Einsatz der besagten Sternpolymere bereits während des Färbens der keratinhaltigen Faser, die Farbechtheit der Färbung, insbesondere die Waschechtheit der Färbung, verbessert. Durch mehrmaliges Waschen der Faser entsteht entlang der keratinischen Faser vom Ansatz bis in die Spitzen keine ungleichmäßige Färbung durch ungleichmäßiges Auswaschen der Farbe. Die Färbung behält ihre gute Egalisierung.

Unter keratinhaltigen Fasern sind erfindungsgemäß Pelze, Wolle, Federn und insbesondere menschliche Haare zu verstehen.

Die kosmetische Verwendung der vorliegenden Erfindung wird verbessert, in dem das mindestens eine besagte Sternpolymer in einem kosmetischen Träger eingearbeitet wird. Die nachfolgend benannten kosmetischen Mittel zur Färbung keratinhaltiger Fasern des zweiten Erfindungsgegenstandes werden daher bevorzugt verwendet.

Ein zweiter Gegenstand der Erfindung ist folglich ein kosmetisches Mittel zur Färbung keratinhaltiger Fasern, insbesondere menschlicher Haare, enthaltend in einem kosmetischen Träger

(a) mindestens ein Sternpolymer, umfassend ein als Zentrum fungierendes Strukturfragment und mindestens drei radial an dieses Zentrum gebundene Gruppen, wobei diese Gruppen

(i) jeweils mindestens eine Polyetherstruktureinheit aufweisen, die eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) jeweils mindestens einen Rest aufweisen, enthaltend

- mindestens eine Gruppe *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht, und/oder
- mindestens eine anionische Gruppe und/oder
- mindestens ein quaternisiertes Stickstoffatom,

und

(b) mindestens eine farbgebende Verbindung,

wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird.

Wird in einer Darstellung einer Strukturformel eine chemische Bindung mit dem Symbol * gekennzeichnet, steht dies für eine freie Valenz des entsprechenden Strukturfragments.

Unter Polymeren werden erfindungsgemäß chemische Verbindungen verstanden, die ein Molekulargewicht von wenigstens 5000 g/mol besitzen. Polymere sind aus einer Vielzahl von Molekülen aufgebaut, in denen eine Art oder mehrere Arten von Atomen oder Atomgruppierungen (sogenannte konstitutive Einheiten, Grundbausteine oder Wiederholungseinheiten) wiederholt aneinander gereiht sind.

Unter "Polyetherstruktureinheit" wird ein chemisches Strukturfragment verstanden, das eine Polyoxyalkylenkette aus Ethylenoxideinheiten oder aus Ethylenoxid- und Propylenoxid-Einheiten umfasst, mit einem maximalen Anteil von 50 Gew.-% an Propylenoxid-Einheiten, bezogen auf das Gewicht der Polyoxyalkylenkette. Die Polyetherstruktureinheit ist gegebenenfalls direkt oder gegebenenfalls über eine chemische Bindung eines vermittelnden Strukturfragments an besagtes Zentrum kovalent gebunden. Als Gerüstmoleküle dienen in diesem Fall bevorzugt organische Verbindungen oder anorganische Metalloxide. Erstere bilden mit der Polyetherstruktureinheit/den Polyetherstruktureinheiten eine Polyether modifizierte organische Verbindung. Letztere bilden mit der Polyetherstruktureinheit/den Polyetherstruktureinheiten einen Polyether modifizierten Feststoffpartikel. Unter "organische Verbindungen" werden chemische Verbindungen basierend auf einem Kohlenwasserstoff-Strukturfragment verstanden. Entsprechende Kohlenwasserstoffstrukturfragmente leiten sich von linearen, verzweigten, zyklischen oder heterozyklischen Kohlenwasserstoffen ab, wobei all diese jeweils gesättigt, ungesättigt oder aromatisch sein dürfen.

Unter "Polyether modifizierte organische Verbindungen" werden erfindungsgemäß organische Verbindungen verstanden, die mit mindestens einer Polyetherstruktureinheit modifiziert sind, wobei die Polyetherstruktureinheit jeweils eine chemische Bindung zu der zu modifizierenden organischen Verbindung ausbilden.

Unter "Feststoffpartikel" werden alle bei Raumtemperatur partikulär vorliegenden Feststoffe verstanden.

Unter "Polyether modifizierter Feststoffpartikel" werden erfindungsgemäß Feststoffpartikel verstanden, die an der Oberfläche mit Polyethern modifiziert sind, wobei die Polyether eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette.

Unter einer Ethylenoxid-Einheit ist im Sinne der vorliegenden Erfindung eine Einheit der allgemeinen Formel (1)

$$*\text{-}CH_2\text{-}CH_2\text{-}O\text{-}* \qquad \text{Formel (1)}$$

zu verstehen, und unter einer Propylenoxid-Einheit ist im Sinne der vorliegenden Erfindung eine Einheit der allgemeinen Formel (2)

$$*\text{-}CH_2\text{-}CH(CH_3)\text{-}O\text{-}* \qquad \text{Formel (2)}$$

zu verstehen.

Enthält die Polyoxyalkylenkette der Polyetherstruktureinheit Ethylenoxid- und Propylenoxid-Einheiten so beträgt der maximale Anteil an Propylenoxid-Einheiten in der erfindungsgemäßen Polyetherstruktureinheit der besagten Sternpolymere vorzugsweise 40 Gew.-% und besonders bevorzugt maximal 30 Gew.-%, bezogen auf das Gewicht der Polyoxyalkylenkette.

Erfindungsgemäß bevorzugte Sternpolymere weisen bevorzugt als radial an das Zentrum gebundene Reste mindestens drei Reste mit der Struktureinheit *-K'-A-K-T auf, worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung oder aus einem Molekülfragment mit zwei freien Valenzen,

T steht für ein Strukturfragment, umfassend

- mindestens eine Gruppe $*\text{-}Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht, oder
- mindestens eine anionische Gruppe, oder
- mindestens ein quaternisiertes Stickstoffatom.

Dabei ist es wiederum bevorzugt, wenn A für ein Strukturfragment der Formel (A1) steht

$$*\text{-}(OCH_2CH_2)_n\text{-}(OCH_2CH(CH_3))_m\text{-}* \qquad \text{(A1)}$$

worin

n eine ganze Zahl von 1 bis 500 bedeutet,
m eine ganze Zahl von 0 bis 500 bedeutet und
das Strukturfragment der Formel (A1) einen maximalen Anteil von 50 Gew.-% an Propylenoxid-Einheiten, bezogen auf das Gewicht des Strukturfragments (A1) hat. Die Ethylenoxid- und Propylenoxid-Einheiten gemäß Formel (PE-1) bzw. gemäß Formel (A1) können statistisch verteilt sein oder gradientenartig verteilt sein oder in mindestens zwei Blöcken vorliegen.

Bevorzugte Reste *-K'-A-K-T sind solche, in denen die Reste K und K' unabhängig voneinander für eine kovalente Bindung, eine Oxygruppe, eine Iminogruppe, eine ($C_1$ bis $C_6$)-Alkylengruppe oder mindestens eine der folgenden

Konnektivitäten (K1) bis (K10) stehen

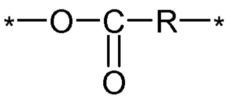 (K1) 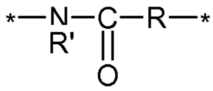 (K2) 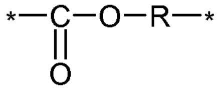 (K3)

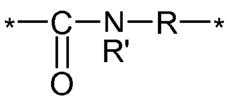 (K4) 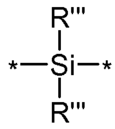 (K5) 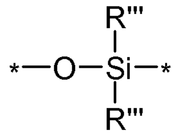 (K6)

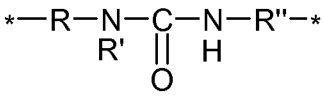 (K7) 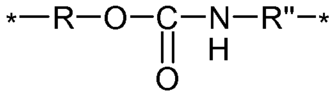 (K8)

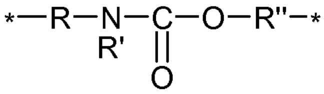 (K9) 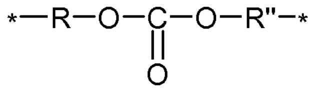 (K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,

R' ein Wasserstoffatom oder eine ($C_1$ bis $C_4$)-Alkylgruppe bedeutet,

R'" unabhängig voneinander eine ($C_1$ bis $C_4$)-Alkylgruppe oder eine Arylgruppe bedeutet.Bevorzugte kosmetische Träger sind wasserhaltige kosmetische Träger, alkoholische kosmetische Träger oder wässrig-alkoholische kosmetische Träger.

Besonders bevorzugt steht T des Rests *-K'-A-K-T für ein Strukturfragment, umfassend

- mindestens eine Gruppe *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht,
  oder
- mindestens eine anionische Gruppe.

Besagte Gruppe *-Si(OR)$_x$(R')$_{3-x}$ ist eine ganz besonders bevorzugte Gruppe des Strukturfragments T.

Zum Zwecke der Färbung keratinhaltiger Fasern sind solche Träger beispielsweise Lotionen, Wasser-in-Öl-Emulsionen, Öl-in-Wasser-Emulsionen, Cremes, Gele, Schäume oder andere Zubereitungen, die für die Anwendung auf dem Haar geeignet sind.

Unter wässrig-alkoholischen Trägern sind im Sinne der vorliegenden Erfindung wässrige Zusammensetzungen enthaltend 3 bis 70 Gew.-% eines organischen Lösemittels, insbesondere eines $C_1$-$C_7$-Alkohols (bevorzugt Ethanol, Isopropanol, Glyzerin, Benzylalkohol, Ethyldiglykol, 1,2-Propylenglykol oder 1,3-Propylenglykol, Methoxybutanol,) zu verstehen. Bevorzugt sind dabei alle wasserlöslichen organischen Lösemittel.

Die besagten Sternpolymere sind in den erfindungsgemäßen Mitteln bevorzugt in einer Menge von 0,01 bis 15,0 Gew.-%, besonders bevorzugt von 0,1 bis 8,0 Gew.-%, ganz besonders bevorzugt von 0,2 bis 5,0 Gew.-%, am bevorzugtesten von 0,25 bis 2,5 Gew.-%, jeweils bezogen auf das Gewicht des gesamten Mittels, enthalten.

Es ist erfindungsgemäß ebenso bevorzugt, die besagten Sternpolymere der Komponente (a) und die farbgebenden Verbindungen der Komponente (b) in den erfindungsgemäßen Mitteln in einem Gewichtsverhältnisbereich von 10 zu 1 bis 1 zu 15, bevorzugt von 3 zu 1 bis 1 zu 10, besonders bevorzugt von 1 zu 1 bis 1 zu 8 , einzusetzen.

Erfindungsgemäß bevorzugte kosmetische Mittel enthalten als besagtes Sternpolymer mindestens eine Verbindung der allgemeinen Formel (PE-1)

$$[Q]-(K'\text{-}A\text{-}K\text{-}T)_n \qquad \text{(PE-1)}$$

worin

| | |
|---|---|
| A | eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A, |
| K und K' | stehen unabhängig voneinander für eine Konnektivität, ausgewählt aus einer kovalenten Bindung oder aus einem Molekülfragment mit zwei freien Valenzen, |
| T | steht für ein Strukturfragment, umfassend<br><br>•mindestens eine Gruppe *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht, oder<br><br>•mindestens eine anionische Gruppe, oder<br><br>•mindestens ein quaternisiertes Stickstoffatom, |
| Q | bedeutet ein als Zentrum fungierendes, organisches Strukturfragment, abgeleitet von linearen, verzweigten, zyklischen oder heterozyklischen Kohlenwasserstoffen, wobei all diese jeweils gesättigt, ungesättigt oder aromatisch sein dürfen, |
| n | steht für eine ganze Zahl von 3 bis 64 (insbesondere für 3, 4, 5, 6, 7 oder 8). |

A steht gemäß Formel (PE-1) bevorzugt für ein Strukturfragment der Formel (A1)

$$*\text{-}(OCH_2CH_2)_p\text{-}(OCH_2CH(CH_3))_m\text{-}* \qquad \text{(A1)}$$

worin

p eine ganze Zahl von 1 bis 500 bedeutet,
m eine ganze Zahl von 0 bis 500 bedeutet und

das Strukturfragment der Formel (A1) einen maximalen Anteil von 50 Gew.-% an Propylenoxid-Einheiten, bezogen auf das Gewicht des Strukturfragments (A1) hat. Die Ethylenoxid- und Propylenoxid-Einheiten gemäß Formel (PE-1) bzw. gemäß Formel (A1) können statistisch verteilt sein oder gradientenartig verteilt sein oder in mindestens zwei Blöcken vorliegen.

Steht die Gruppe A der Verbindungen nach Formel (PE-1) (bzw. nach allen nachfolgenden Formeln enthaltend die Gruppe A) für eine Polyoxyalkylenkette aus Ethylenoxid- und Propylenoxid-Einheiten so beträgt der maximale Anteil an Propylenoxid-Einheiten vorzugsweise 40 Gew.-% und besonders bevorzugt maximal 30 Gew.-%, bezogen auf das Gewicht von A.

Die Reste K und K' gemäß Formel (PE-1) stehen bevorzugt unabhängig voneinander für eine kovalente Bindung, eine Oxygruppe, eine ($C_1$ bis $C_6$)-Alkylengruppe, eine Iminogruppe oder mindestens eine der folgenden Konnektivitäten (K1) bis (K10)

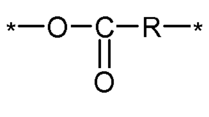
(K1)

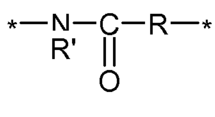
(K2)

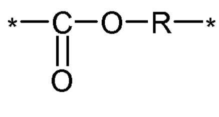
(K3)

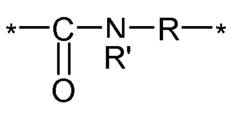
(K4)

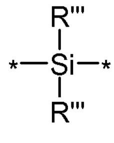
(K5)

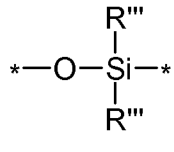
(K6)

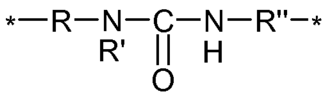 (K7) 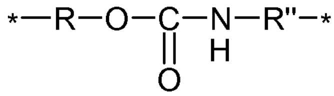 (K8)

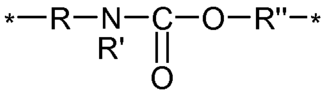 (K9) 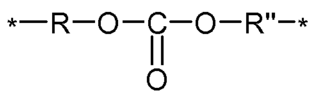 (K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,
R' ein Wasserstoffatom oder eine ($C_1$ bis $C_4$)-Alkylgruppe bedeutet,
R'" unabhängig voneinander eine ($C_1$ bis $C_4$)-Alkylgruppe oder eine Arylgruppe bedeutet.

T steht gemäß Formel (PE-1) bevorzugt für einen Rest $*\text{-Si(OR)}_x(\text{R'})_{3\text{-}x}$, worin R und R' unabhängig voneinander für eine ($C_1$ bis $C_4$)-Alkylgruppe (insbesondere für Methyl oder Ethyl) stehen und x für 2 oder 3 steht (bevorzugt für Triethoxysilyl).
Ganz besonders bevorzugt steht das Strukturfragment -K-T gemäß Formeln (PE-1) für eine Gruppe

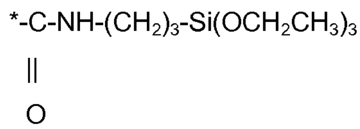

Der Rest Q der Formel (PE-1) steht bevorzugt für ein organisches Strukturfragment mit 2 bis 30 Kohlenstoffatomen, besonders bevorzugt mit 2 bis 20 Kohlenstoffatomen.
Q wird bevorzugt abgeleitet von Glyzerin, Monosaccharid, Disaccharid. Ganz besonders bevorzugt leitet sich Q von einer Verbindung ab, ausgewählt aus Sorbitol, 1,5-Anhydro-Sorbitol, 1,4-Anhydrosorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Polyglycerin-3, Glucose, Fructose, Galactose, Ribose, Xylose, Mannose, Saccharose, Cellobiose, Gentiobiose, Isomaltose, Lactose, Lactulose, Maltose, Maltutose, Melibiose, Trehalose, Nigerose, Palatinose, Rutinose, Arabinose.
Ganz besonders bevorzugte Sternpolymere werden ausgewählt aus mindestens einer Verbindung der Formel (PE-1a) oder (PE-1b) oder (PE-1c) oder (PE-1d) oder (PE-1e) oder (PE-1f) oder Mischungen daraus,

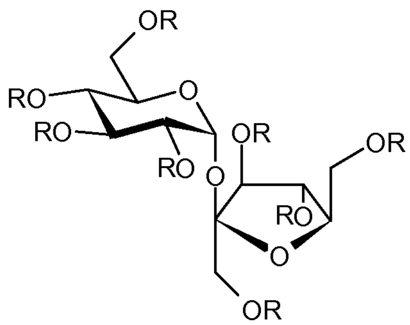

(PE-1a)

worin
mindestens drei Gruppen R für eine Gruppe $*\text{-}(CH_2CH_2O)_p\text{-}(CHCH_3CH_2O)_m\text{-K-T}$ stehen und die übrigen Gruppen R für ein Wasserstoffatom oder eine Gruppe *-K-T stehen, worin unabhängig voneinander
p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

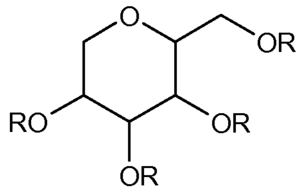

(PE-1b)

worin

mindestens drei Gruppen R für eine Gruppe *-$(CH_2CH_2O)_p$-$(CHCH_3CH_2O)_m$-K-T stehen und die übrigen Gruppen R für ein Wasserstoffatom oder eine Gruppe *-K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

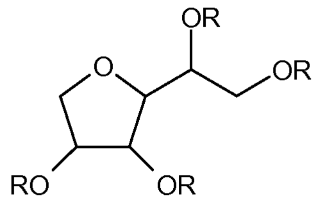

(PE-1c)

worin

mindestens drei Gruppen R für eine Gruppe *-$(CH_2CH_2O)_p$-$(CHCH_3CH_2O)_m$-K-T stehen und die übrigen Gruppen R für ein Wasserstoffatom oder eine Gruppe *-K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

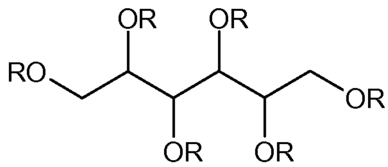

(PE-1d)

worin

mindestens drei Gruppen R für eine Gruppe -$(CH_2CH_2O)_p$-$(CHCH_3CH_2O)_m$-K-T stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis

zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Rest $*\text{-Si(OR)}_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

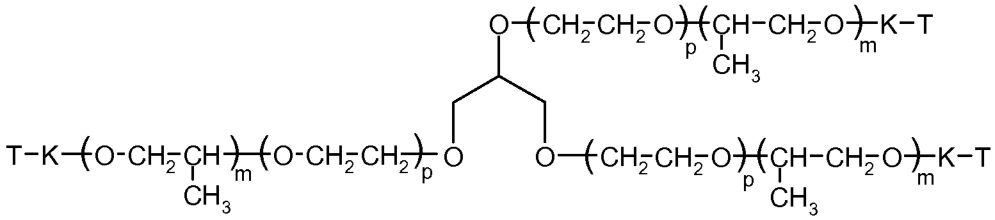

(PE-1f)

worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, hat;

K stehen unabhängig voneinander für eine Konnektivität, ausgewählt aus einer kovalenten Bindung oder aus einem Molekülfragment mit zwei freien Valenzen,

T stehen unabhängig voneinander für ein Molekülfragment umfassend mindestens einen Rest $*\text{-Si(OR)}_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht.

Die Ethylenoxid- und Propylenoxid-Einheiten können statistisch verteilt sein oder gradientenartig verteilt sein oder in mindestens zwei Blöcken vorliegen.

Es gelten für die Formeln (PE-1a) bis (PE-1f) die zuvor bevorzugten Gruppen K und T. Ganz besonders bevorzugt steht das Strukturfragment -K-T gemäß Formeln (PE-1a) bis (PE-1f) für eine Gruppe

$*\text{-C-NH-}(CH_2)_3\text{-Si}(OCH_2CH_3)_3$

||

O

Die Sternpolymere der Formel (PE-1) sind in den erfindungsgemäßen Mitteln bevorzugt in einer Menge von 0,01 bis 15,0 Gew.-%, besonders bevorzugt von 0,1 bis 8,0 Gew.-%, ganz besonders bevorzugt 0,2 bis 5,0 Gew.-%, am bevorzugtesten von 0,25 bis 2,5 Gew.-%, jeweils bezogen auf das Gewicht des gesamten Mittels, enthalten.

Es werden erfindungsgemäß solche kosmetischen Mittel bevorzugt, enthaltend in einem kosmetisch akzeptablen Träger

(a) Sternpolymere, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit

(ii) mindestens 3 Moläquivalenten mindestens eines Polyethers der Formel (I) erhalten werden

T-K-A-K'-Y (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen

auf das Gewicht von A,

K und K' stehen unabhängig voneinander für eine Konnektivität, ausgewählt aus einer kovalenten Bindung oder aus einem Molekülfragment mit zwei freien Valenzen,

T steht für

- eine Gruppe *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht, oder
- eine anionische Gruppe, oder
- ein quaternisiertes Stickstoffatom,

Y für eine gegenüber eine Hydroxygruppe oder Aminogruppe reaktive Gruppe steht,

und

(b) mindestens eine farbgebende Verbindung.

Die Angabe der Moläquivalente bezieht sich auf die Stoffmenge der eingesetzten organischen Verbindung.

Die organische Verbindung mit mindestens drei besagten Resten (vgl. (a) (i)) wird bevorzugt ausgewählt aus Glyzerin, Monosaccharid, Disaccharid. Besonders bevorzugt wird besagte organische Verbindung ausgewählt aus Sorbitol, 1,5-Anhydro-Sorbitol, 1,4-Anhydrosorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Polyglycerin-3, Glucose, Fructose, Galactose, Ribose, Xylose, Mannose, Saccharose, Cellobiose, Gentiobiose, Isomaltose, Lactose, Lactulose, Maltose, Maltutose, Melibiose, Trehalose, Nigerose, Palatinose, Rutinose, Arabinose.

Für die Reste A, K, K' und T gelten die unter Formel (PE-1) genannten, bevorzugten Ausführungsformen.

Bevorzugt steht der Rest Y gemäß Formel (I) für

- eine Isocyanatfunktion, (die Isocyanatfunktion ist besonders bevorzugt)
- eine Gruppe *-Si(OR)$_x$(R')$_{3-x}$
  worin R und R' unabhängig voneinander für eine ($C_1$ bis $C_4$)-Alkylgruppe (insbesondere für Methyl oder Ethyl) stehen und x für 1, 2 oder 3 steht,
- eine Gruppe *-Si(R)$_x$(R')$_{3-x}$
  worin R für ein Halogenatom steht (bevorzugt für Chlor oder Brom) und R' unabhängig voneinander für eine ($C_1$ bis $C_4$)-Alkylgruppe (insbesondere für Methyl oder Ethyl) stehen und x für 1, 2 oder 3 steht,
- eine Gruppe T-K-C(O)-O-C(O)-*, worin T und K gemäß Formel (I) definiert sind,
- eine Gruppe *-C(O)-Hal, worin Hal für Chlor oder Brom steht,
- eine Epoxygruppe,
- eine Formylgruppe.

Die Sternpolymere dieser Ausführungsform sind in den erfindungsgemäßen Mitteln bevorzugt in einer Menge von 0,01 bis 10,0 Gew.-%, besonders bevorzugt von 0,1 bis 2,0 Gew.-%, ganz besonders bevorzugt 0,2 bis 1,0 Gew.-%, jeweils bezogen auf das Gewicht des gesamten Mittels, enthalten.

Die Polyether der Formel (I) besitzen bevorzugt eine Molmasse von 1 bis 200 kDa, besonders bevorzugt von 1 bis 10 kDa.

Die im Rahmen der Erfindung zur Herstellung der Sternpolymere einsetzbaren Polyether der Formel (I) sind erhältlich durch Umsetzung von mindestens einer Verbindung der Formel (II):

X-A-X' (II)

worin

A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,

X und X' unabhängig voneinander für ein Strukturfragment stehen, enthaltend eine Gruppe OH, $NH_2$ oder NHR

mit mindestens 2 Moläquivalenten einer oder mehrerer Verbindungen der Formel (III)

Y-K-T (III)

worin,

Y für eine gegenüber -OH, $-NH_2$, -NHR, $-NR_2$ reaktive Gruppe steht,

K steht für eine Konnektivität, ausgewählt aus einer kovalenten Bindung oder aus einem Molekülfragment mit zwei freien Valenzen,

T für ein Molekülfragment steht, umfassend

- eine Gruppe $*\text{-Si(OR)}_x(\text{R'})_{3-x}$, worin R und R' unabhängig voneinander für eine ($C_1$ bis $C_4$)-Alkylgruppe (insbesondere für Methyl oder Ethyl) stehen, x für 1, 2 oder 3 steht, oder
- eine anionische Gruppe, oder
- ein quaternisiertes Stickstoffatom,

Die Angabe der Moläquivalente bezieht sich auf die Stoffmenge der eingesetzten Verbindung(en) der Formel (II).

Gemäß obigem Herstellverfahren zur Herstellung der Verbindungen der Formel (I) werden als Verbindungen der Formel (II) beispielsweise dihydroxyterminierte Polyoxyalkylen-Diole, diaminoterminierte Polyoxyalkylen-Diamine, monohydroxy-monoamin-terminierte Polyoxyalkylen-Monol-Monoamine, monohydroxy-monoalkoxy-terminierte Polyoxyalkylen-Monole oder monoamino-monoalkoxy-terminierte Polyoxyalkylen-Monoamine eingesetzt, worunter die Diamine und Diole bevozugt sind.

Vorzugsweise stehen die Reste X und X' der Formel (II) unabhängig voneinander für OH, $NH_2$ und NHR, besonders bevorzugt für OH und $NH_2$.

Bevorzugt steht der Rest R in den Gruppen NHR und $NR_2$ der Formel (III) für eine lineare oder verzweigte Alkylgruppen mit 1 bis 10, vorzugsweise 1 bis 6 Kohlenstoffatomen.

Das zahlenmittlere Molekulargewicht der Verbindung der Formel (II) beträgt vorzugsweise 500 bis 30000 g/mol, besonders bevorzugt 1000 bis 20000, besser noch 2000 bis 18000 g/mol, und lässt sich beispielsweise durch Endgruppenbestimmung ermitteln.

Für die Reste A, K, K' und T gelten die unter Formel (PE-1) genannten, bevorzugten Ausführungsformen.

Im Rahmen einer bevorzugten Ausführungsform (zur Herstellung) der erfindungsgemäßen Sternpolymere umfasst mindestens ein Molekülfragment T der Formel (PE-1), bzw. Formel (I) bzw. Formel (III) mindestens eine anionische Gruppe.

Bei den Molekülfragmenten T der Formel (I) bzw. Formel (III) mit mindestens einer anionischen Gruppe handelt es sich erfindungsgemäß vorzugsweise um ein Molekülfragment, das 1 bis 5, vorzugsweise 3, 4 oder 5, deprotonierbare Säuregruppen umfasst. Die anionischen Gruppen bzw. die deprotonierbaren Säuregruppen der besagten Molekülfragmente T der Formel (I) bzw. Formel (III) werden bevorzugt ausgewählt aus Carboxylgruppe und/oder Sulfonsäuregruppe und/oder Phosphat bzw. deren jeweiligen Salzformen (insbesondere Carboxylgruppe und/oder Sulfonsäuregruppe bzw. deren jeweiligen Salzformen, besonders bevorzugt Carboxylgruppe bzw. deren Salzform).

In einer besonders bevorzugten Ausführungsform enthält das Molekülfragment T der Formel (I) bzw. Formel (III) mindestens eine, vorzugsweise mindestens zwei, besonders bevorzugt 1 bis 5, vor allem 2 bis 5, insbesondere 2, 3, 4 oder 5 Carboxymethyl-Einheiten. In einer ganz besonders bevorzugten Ausführungsform handelt es sich bei dem besagten Molekülfragment um eine Ethylendiamintriacetat-Einheit, die über eines ihrer Stickstoff-Atome kovalent an die Konnektivität K der Formel (I) bzw. Formel (III) gebunden ist.

In einer erfindungsgemäß bevorzugten Ausführungsform zur Herstellung der erfindungsgemäßen Sternpolymere handelt es sich bei dem Molekülfragment T der Formel (PE-1) bzw. der Formel (I) bzw. Formel (III) um eine Silyl-Gruppe der allgemeinen Formel (IV)

$$-CR^a_2\text{-Si}(OR^b)_r(R^c)_{3-r}, \qquad \text{(IV)},$$

wobei

$R^a$ für Wasserstoff oder $C_{1-6}$-Alkyl steht,

$R^b$ für $-\text{Si}(R^d)_t(R^e)_{3-t}$ steht,

$R^c$ für ($C_1$ bis $C_6$)-Alkyl, ($C_1$ bis $C_6$)-Alkoxy oder Hydroxy, vorzugsweise für ($C_1$ bis $C_6$)-Alkoxy oder Hydroxy, steht,

$R^d$ für eine negativ geladene Gruppe steht,

$R^e$ für ($C_1$ bis $C_6$)-Alkyl, $C_{1-6}$-Alkoxy oder Hydroxy, vorzugsweise für ($C_1$ bis $C_6$)-Alkoxy oder Hydroxy, steht,

r für eine Zahl von 1 bis 3, vorzugsweise für 1, steht,

t für eine Zahl von 1 bis 3, vorzugsweise für 1, steht.

$R^d$ steht vorzugsweise für eine Einheit, die 1-5, vorzugsweise 3, 4 oder 5, Säuregruppen, insbesondere Carbonsäuregruppen, umfasst.
Besonders bevorzugt steht $R^d$ der Formel (IV) für eine Gruppe

$$*\text{-B-[(N(CH}_2\text{COOM)-B')}_y\text{-N(CH}_2\text{COOM)}_2]$$

wobei

- B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- M unabhängig voneinander für ein Wasserstoffatom oder ein Äquivalent eines ein- oder mehrwertigen Kations,
- y 1 oder 2 (vorzugsweise 1) bedeutet.

Wenn die obige Gruppe als Säure vorliegt, bedeutet der Rest M ein Wasserstoffatom. Die Fragmente -COOH bilden in diesem Fall eine Carboxylgruppe. Wenn die obige Gruppe in ihrer Salzform vorliegt (Carboxylat), steht M für ein Äquivalent eines ein- oder mehrwertigen Kations. Das ein oder mehrwertige Kation $M^{z+}$ mit einer Ladungszahl z von eins oder höher dient lediglich aus Gründen der Elektroneutralität zur Kompensation der einfach negativen Ladung des bei Salzbildung vorliegenden Carboxylats. Das dafür zu verwendende Äquivalent des entsprechenden Kations beträgt 1/z. Das Fragment -COOM steht im Fall der Salzbildung für die Gruppe:

$$\text{-COO}^- \ 1/z\ (M^{z+})$$

Als ein- oder mehrwertige Kationen $M^{z+}$ kommen prinzipiell alle Kationen in Frage, die physiologisch verträglich sind. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der obigen Gruppe $R^d$ in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.
Ein ganz besonders bevorzugtes Molekülfragment T der Formel (PE-1) bzw. Formel (I) bzw. Formel (III) zur Herstellung der erfindungsgemäßen Sternpolymere folgt der allgemeinen Formel

$$*\text{-B-[(N(CH}_2\text{COOM)-B')}_y\text{-N(CH}_2\text{COOM)}_2]$$

wobei

- B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,
- M unabhängig voneinander für ein Wasserstoffatom oder ein Äquivalent eines ein- oder mehrwertigen Kations,
- y 1 oder 2 (vorzugsweise 1) bedeutet.

Für den Rest M der obigen Formel gilt das zuvor Gesagte (*vide supra*).
Besonders bevorzugte Mittel dieser Ausführungsform weisen Sternpolymere auf, die mindestens einen Polyether mit mindestens einem Molekülfragment T der besagten allgemeinen Formel

$$*\text{-B-[(N(CH}_2\text{COOM)-B')}_y\text{-N(CH}_2\text{COOM))}_2]$$

enthalten.
Besonders bevorzugte Molekülfragmente T aus Formel (PE-1) bzw. Formel (I) bzw. Formel (III) mit der Formel $*\text{-B-[(N(CH}_2\text{COOM)-B')}_y\text{-N(CH}_2\text{COOM))}_2]$ werden ausgewählt aus mindestens einem Vertreter der Gruppe, die gebildet wird aus 3-N-Carboxylmethyl-N-(2'-N',N'-di(carboxymethylamino)ethyl)-aminopropyl (B=Propan-1,3-diyl, B'=Ethan-1,2-diyl, y=1, M wie oben), 3-N-Carboxylmethyl-N-(2'-N',N'-di(carboxymethylamino)ethyl)-N"-carboxymethylamino-ethyl)aminopropyl (B=Propan-1,3-diyl, B'=Ethan-1,2-diyl, y=2, M wie oben). Dabei ist 3-N-Carboxylmethyl-N-(2'-N',N'-di(carboxymethylamino)-ethyl)aminopropyl ganz besonders bevorzugt.

Ganz besonders bevorzugte Verbindungen zur Herstellung der erfindungsgemäßen Sternpolymere verwendbare Verbindungen werden ausgewählt aus mindestens einer Verbindung der Formel (I-1c)

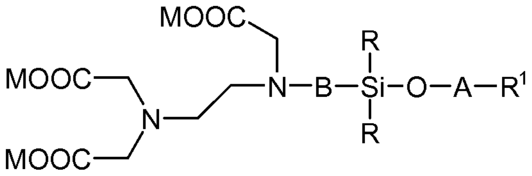

(I-1c)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,

B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,

M unabhängig voneinander für ein Wasserstoffatom oder ein Äquivalent eines ein- oder mehrwertigen Kations,

$R^1$ für Gruppe $R^3_{3-x}(R^2O)_xSi$-K- steht worin

$R^2$ und $R^3$ unabhängig voneinander stehen für eine ($C_1$ bis $C_4$)-Alkylgruppe (insbesondere Methyl oder Ethyl), x eine ganze Zahl 1, 2 oder 3 (insbesondere 3) bedeutet,
K steht für eine Konnektivität, ausgewählt aus einer kovalenten Bindung oder aus einem Molekülfragment mit zwei freien Valenzen,

R unabhängig voneinander für eine ($C_1$ bis $C_4$)-Alkylgruppe (insbesondere für Methyl oder Ethyl) oder eine ($C_1$ bis $C_6$)-Alkoxygruppe (insbesondere Methoxy oder Ethoxy) steht.

Diese wird als Verbindung der Formel (III) bevorzugt mit Polyether-Polyolen gemäß zuvor beschriebenem Verfahren zu den erfindungsgemäßen Polyether modifizierten organischen Verbindungen umgesetzt.

Für die oben genannte Formeln (I-1c) gelten die bevorzugten Ausführungsformen bezüglich der zuvor genannten bevorzugten Merkmale für A, B, B', M, $R^1$ und R *mutatis mutandis.*

Bevorzugt werden im Rahmen dieser Ausführungsform als Verbindung der Formel (III) mindestens eine Verbindung der allgemeinen Formel (III-1) eingesetzt

$$Y\text{-}K\text{-}Si(OR)_xR'_{3-x} \qquad \text{(III-1)}$$

worin,

Y für eine gegenüber OH, $NH_2$, NHR und/oder $NR_2$ reaktive Gruppe (insbesondere für eine Isocyanatgruppe, ein Halogenatom, eine Carbonsäureanhydridgruppe, eine Halogencarbonylgruppe (insbesondere Chlorcarbonyl), eine Epoxygruppe, eine Formylgruppe), steht,

K wie unter Formel (I) (bzw. wie die vorgenannten bevorzugten Konnektivitäten) definiert ist,

R für eine ($C_1$ bis $C_4$)-Alkylgruppe oder eine ($C_2$ bis $C_4$)-Acylgruppe (insbesondere für Ethyl oder Methyl) steht,

R' für eine ($C_1$ bis $C_4$)-Alkylgruppe (insbesondere Methyl oder Ethyl) steht,

x für 1, 2 oder 3 steht.

Zu den Verbindungen der allgemeinen Formel (III-1) gehören alle funktionellen Silan-Derivate, die zur Reaktion gegenüber X-Gruppen der Formel (III-1) befähigt sind. Beispiele sind Acrylat-Silane wie (3-Acryloxypropyl)trimethoxysilan, (Acryloxymethyl)triethoxysilan und (Acryloxymethyl)methyl-dimethoxysilan, Isocyanato-Silane wie (3-Isocyanatopropyl)trimethoxysilan, (3-Isocyanatopropyl)triethoxysilan, (Isocyanatomethyl)methyl-Dimethoxysilan und (Isocyanatomethyl)trimethoxysilan, Aldehyde-Silane wie Triethoxysilylundecanal und Triethoxysilylbutyraldehyde, Epoxy-Silane wie (3-Glycidoxypropyl)trimethoxysilan, Anhydridsilane wie 3-(Triethoxysilyl)propylbernsteinsäureanhydrid, Halogen-Silane wie Chloromethyltrimethoxysilan, 3-Chloropropylmethyldimethoxysilan, sowie Tetraethylsilikat (TEOS), die kommerziell beispielsweise bei der Wacker Chemie GmbH (Burghausen), der Gelest, Inc. (Morrisville, USA) oder ABCR GmbH & Co. KG (Karlsruhe) erhältlich sind oder nach bekannten Verfahren hergestellt werden können. Besonders bevorzugt werden Isocyanato-Silane bzw. Anhydrid-Silane. Bei einer vollständigen Umsetzung aller Hydroxyenden mit Isocyanatosilanen erhält man komplett silylierte Polyether. Die Gruppe K enthält in einem solchen Fall nur die Atomgruppe, die im Ausgangs-Isocyanatosilan zwischen der Isocyanatogruppe und der Silylgruppe steht. Bei einer vollständigen Umsetzung aller Hydroxyenden mit Anhydrid-Silanen, beispielsweise 3-(Triethoxysilyl)propylbernsteinsäureanhydrid, erhält man ebenfalls komplett silylierte Polyether. Die Gruppe K' enthält in einem solchen Fall nur die Atomgruppe, die im Ausgangs-Anhydridsilan zwischen der Anhydridgruppe und der Silylgruppe steht.

Stehen die Reste X und X' in der allgemeinen Formel (II) für OH, $NH_2$ oder NHR, so erfolgt die Reaktion mit den Verbindungen der allgemeinen Formel (III) bzw. (III-1) üblicherweise entweder unter Abspaltung der Verbindung HY - wie beispielsweise im Falle der Umsetzung einer OH-Gruppe mit einem Monohalogensilan (K = direkte Bindung) - oder aber unter Addition - wie beispielsweise im Falle der Umsetzung einer OH-Gruppe mit einem Isocyanatoalkylsilan (Bildung eines Urethans).

Vorzugsweise stehen die Reste X und X' der Formel (II) unabhängig voneinander für OH, $NH_2$ und NHR, besonders bevorzugt für OH und $NH_2$.

Bevorzugt steht der Rest R in den Gruppen NHR, $NR_2$ und OR der Formel (III-1) für eine lineare oder verzweigte Alkylgruppen mit 1 bis 10, vorzugsweise 1 bis 6 Kohlenstoffatomen.

Bei der Reaktion zwischen den Verbindungen der Formel (I) und den Verbindungen der Formel (II) wird mindestens ein Wasserstoffatom, vorzugsweise werden bis zu vier Wasserstoffatome der OH- und/oder $NH_2$-Gruppen mit je einem Molekül der Verbindung der allgemeinen Formel (II) umgesetzt, so dass zumindest monosilylierte, im Falle der Diaminoverbindungen der allgemeinen Formel (I) bis zu vierfach silylierte Polyether entstehen.

Ganz besonders bevorzugte Polyether zur Herstellung der erfindungsgemäßen Sternpolymere werden ausgewählt aus mindestens einer Verbindung der Formeln (I-1a) oder (I-1b)

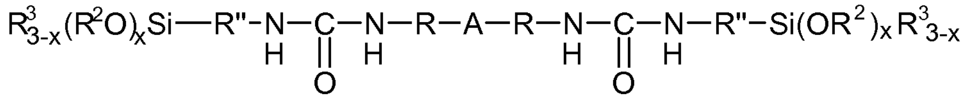

(I-1a)

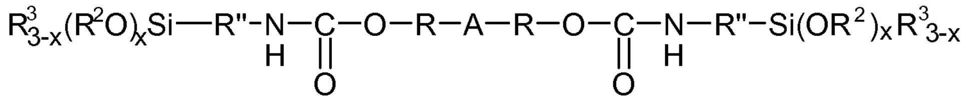

(I-1b)

worin

| | |
|---|---|
| A | eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A, |
| R und R" | unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl, Phenylen, |
| $R^1$ | für eine ($C_1$ bis $C_6$)-Alkylgruppe, ein Wasserstoffatom oder eine Gruppe $R^3_{3-x}(R^2O)_x$Si-K-steht, |
| $R^2$ | für eine ($C_1$ bis $C_4$)-Alkylgruppe (insbesondere für Methyl oder Ethyl) steht, |
| $R^3$ | für eine ($C_1$ bis $C_6$)-Alkylgruppe oder eine Arylgruppe (insbesondere Methyl) steht, |
| x | für 1, 2, 3 (insbesondere 3) steht. |

Im Rahmen einer weiteren Ausführungsform der Erfindung weist das besagte Sternpolymer als Zentrum einen Feststoffpartikel auf, welcher an der Oberfläche mit mindestens drei Resten *-K'-A-K-T modifiziert ist, worin

| | |
|---|---|
| A | eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A, |
| K und K' | stehen unabhängig voneinander für eine Konnektivität, ausgewählt aus einer kovalenten Bindung oder aus einem Molekülfragment mit zwei freien Valenzen, |
| T | steht für ein Strukturfragment, umfassend |

• mindestens eine Gruppe *-Si(OR)$_x$(R')$_{3-x}$, worin R und R' unabhängig voneinander für eine ($C_1$ bis $C_4$)-Al-

kylgruppe (insbesondere für Methyl oder Ethyl) stehen, x für 1, 2 oder 3 steht, oder

• mindestens eine anionische Gruppe, oder

• mindestens ein quaternisiertes Stickstoffatom.

Das Gerüst der Feststoffpartikel wird bevorzugt ausgewählt unter Aluminaten, Silikaten, Aluminiumsilikaten, Zinkoxid, Titandioxid sowie $SiO_2$, insbesondere unter Aluminaten, Silikaten, Aluminiumsilikaten sowie Silikagel. Ebenso erfindungsgemäß sind jeweils Gemische dieser bevorzugten bzw. besonders bevorzugten Feststoffpartikel.

Besonders bevorzugte Aluminate werden ausgewählt unter alpha-Aluminiumoxid, beta-Aluminiumoxid, gamma-Aluminiumoxid sowie deren Gemischen.

Besonders bevorzugte Aluminiumsilikate (auch Alumosilikate genannt) werden ausgewählt unter Phyllosilikaten, Tectosilikaten.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), Zeolithen.

Zeolithe sind natürliche oder synthetische kristalline Aluminiumsilikate von Alkali- oder Erdalkali metallen. Zeolithe bestehen aus $SiO_4$- und $AlO_4$-Tetraedern, die durch vierer, sechser, achter oder zwölfer Sauerstoffringen verbunden sind, wodurch Kavitäten entstehen, die sich durch den gesamten Zeolithkristall erstrecken. Zu den bevorzugten Zeolithen gehören die Zeolithe vom Typ A, K, L, P-L, O, T, X, Y und Ω sowie deren Mischungen. Besonders bevorzugte Zeolithe werden insbesondere ausgewählt unter Zeolith A (hier insbesondere unter $Na_{12}(AlO_2)_{12}(SiO_2)_{12}]$), $Ca_5Na_5[(AlO_2)_{12}(SiO_2)_{12}]$, $[K_{12}[(AlO_2)_{12}(SiO_2)_{12}]$), Zeolith X (hier insbesondere unter $Na_{86}[(AlO_2)_{86}(SiO_2)_{106}]$), $Ca_{40}Na_6[(AlO_2)_{86}(SiO_2)_{106})]$, $Sr_{21}Ba_{22}[(AlO_2)_{86}(SiO_2)_{106}]$), Zeolith Y (hier insbesondere unter $Na_{56}[(AlO_2)_{56}(SiO_2)_{136}]$, $Na_{56}[(AlO_2)_{56}(SiO_2)_{136}]$), ZSM-5 (hier insbesondere $Na_3[(AlO_2)_3(SiO_2)_{93}]$), Mordenit, Silicalit $(SiO_2)_{96}$.

Für die Reste A, K, K' und T gelten die unter Formel (PE-1) genannten, bevorzugten Ausführungsformen.

Es werden erfindungsgemäß solche kosmetischen Mittel bevorzugt, enthaltend in einem kosmetisch akzeptablen Träger

(a) mindestens ein Sternpolymer in Form eines Polyether-modifizierten Feststoffpartikels, welche durch Umsetzung von Hydroxygruppen-haltigen Feststoffpartikeln mit mindestens einem Polyether der Formel (I) erhalten werden

T-K-A-K'-T' (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung oder aus einem Molekülfragment mit zwei freien Valenzen,

T und T' stehen unabhängig voneinander für ein Molekülfragment, umfassend mindestens

- eine Gruppe $*-Si(OR)_{x(}(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht, oder

- eine anionische Gruppe, oder

- ein quaternisiertes Stickstoffatom,

wobei höchstens ein Rest aus T oder T' zusätzlich für eine ($C_1$ bis $C_6$)-Alkylgruppe, eine Arylgruppe, eine Aryl-($C_1$ bis $C_6$)-alkylgruppe, eine ($C_1$ bis $C_6$)-Alkoxygruppe oder eine ($C_2$ bis $C_6$)-Acylgruppe stehen kann,

und

(b) mindestens eine farbgebende Verbindung,

wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird.

Die Sternpolymere in Form eines Polyether-modifizierten Feststoffpartikels sind in den erfindungsgemäßen Mitteln bevorzugt in einer Menge von 0,01 bis 15,0 Gew.-%, bevorzugt von 0,1 bis 8,0 Gew.-%, besonders bevorzugt 0,2 bis 5,0 Gew.-%, am bevorzugtesten von 0,25 bis 2,5 Gew.-%, jeweils bezogen auf das Gewicht des gesamten Mittels, enthalten.

Es ist erfindungsgemäß bevorzugt, als Entwicklerkomponente ein p-Phenylendiaminderivat oder eines seiner physiologisch verträglichen Salze einzusetzen. Besonders bevorzugt sind p-Phenylendiaminderivate der Formel (E1)

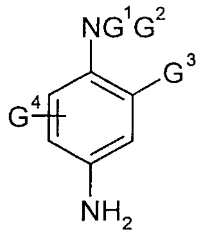

(E1)

wobei

- $G^1$ steht für ein Wasserstoffatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen 4'-Aminophenylrest oder einen ($C_1$ bis $C_4$)-Alkylrest, der mit einer stickstoffhaltigen Gruppe, einem Phenyl- oder einem 4'-Aminophenylrest substituiert ist;
- $G^2$ steht für ein Wasserstoffatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest oder einen ($C_1$ bis $C_4$)-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Hydroxyalkoxyrest, einen ($C_1$ bis $C_4$)-Alkoxy-($C_1$ bis $C_4$)-alkylrest, einen ($C_1$ bis $C_4$)-Acetylaminoalkoxyrest, einen Mesylamino-($C_1$ bis $C_4$)-alkoxyrest oder einen ($C_1$ bis $C_4$)-Carbamoylaminoalkoxyrest;
- $G^4$ steht für ein Wasserstoffatom, ein Halogenatom, einen ($C_1$ bis $C_4$)-Alkylrest oder einen ($C_1$ bis $C_4$)-Alkoxy-($C_1$ bis $C_4$)-alkylrest, oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-Methoxymethyl-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, 2-Methoxymethyl-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

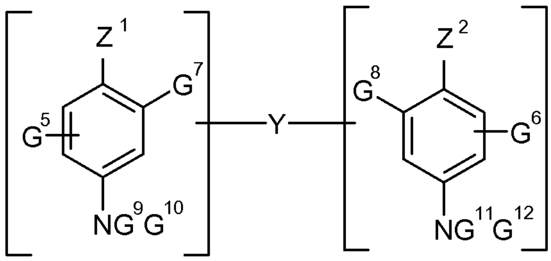

(E2)

wobei:

- $Z^1$ und $Z^2$ stehen unabhängig voneinander für einen Hydroxyl- oder $NH_2$-Rest, der gegebenenfalls durch einen ($C_1$ bis $C_4$)-Alkylrest, durch einen ($C_1$ bis $C_4$)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder ($C_1$ bis $C_8$)-Alkoxyreste substituiert sein kann, oder eine direkte Bindung,
- $G^5$ und $G^6$ stehen unabhängig voneinander für ein Wasserstoff- oder Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen ($C_1$ bis $C_4$)-Alkylrest,

mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin ist es erfindungsgemäß bevorzugt, als Entwicklerkomponente mindestens ein p-Aminophenolderivat oder eines seiner physiologisch verträglichen Salze einzusetzen. Besonders bevorzugt sind p-Aminophenolderivate der Formel (E3)

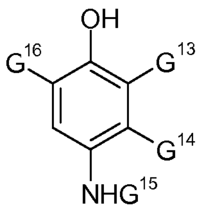

(E3)

wobei:

- $G^{13}$ steht für ein Wasserstoffatom, ein Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen ($C_1$ bis $C_4$)-Aminoalkylrest, einen Hydroxy-($C_1$ bis $C_4$)-alkylaminorest, einen ($C_1$ bis $C_4$)-Hydroxyalkoxyrest, einen ($C_1$ bis $C_4$)-Hydroxyalkyl-($C_1$ bis $C_4$)-aminoalkylrest oder einen (Di-[($C_1$ bis $C_4$)-alkyl]amino)-(C, bis $C_4$)-alkylrest, und
- $G^{14}$ steht für ein Wasserstoff- oder Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen ($C_1$ bis $C_4$)-Aminoalkylrest oder einen ($C_1$ bis $C_4$)-Cyanoalkylrest,
- $G^{15}$ steht für Wasserstoff, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen Phenylrest oder einen Benzylrest, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin wird die Entwicklerkomponente bevorzugt ausgewählt aus heterocyclischen Entwicklerkomponenten, wie beispielsweise aus Pyrimidinderivaten, Pyrazolderivaten, bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen,

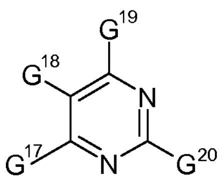

(E4)

worin

- $G^{17}$, $G^{18}$ und $G^{19}$ unabhängig voneinander für ein Wasserstoffatom, eine Hydroxygruppe, eine ($C_1$ bis $C_4$)-Alkoxygruppe oder eine Aminogruppe steht und
- $G^{20}$ für eine Hydroxygruppe oder eine Gruppe $-NG^{21}G^{22}$ steht, worin $G^{21}$ und $G^{22}$ unabhängig voneinander stehen für ein Wasserstoffatom, eine ($C_1$ bis $C_4$)-Alkylgruppe, eine ($C_1$ bis $C_4$)-Monohydroxyalkylgruppe,

mit der Maßgabe, dass maximal zwei der Gruppen aus $G^{17}$, $G^{18}$, $G^{19}$ und $G^{20}$ eine Hydroxygruppe bedeuten und höchstens zwei der Reste $G^{17}$, $G^{18}$ und $G^{19}$ für ein Wasserstoffatom stehen. Dabei ist es wiederum bevorzugt, wenn gemäß Formel (E4) mindestens zwei Gruppen aus $G^{17}$, $G^{18}$, $G^{19}$ und $G^{20}$ für eine Gruppe $-NG^{21}G^{22}$ stehen und höchstens zwei Gruppen aus $G^{17}$, $G^{18}$, $G^{19}$ und $G^{20}$ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5),

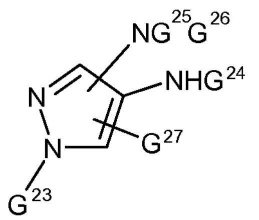

(E5)

worin

- $G^{23}$, $G^{24}$, $G^{25}$ stehen unabhängig voneinander für ein Wasserstoffatom, eine ($C_1$ bis $C_4$)-Alkylgruppe, eine ($C_1$ bis $C_4$)-Monohydroxyalkylgruppe, eine ($C_2$ bis $C_4$)-Polyhydroxyalkylgruppe, eine gegebenenfalls substituierte Arylgruppe oder eine gegebenenfalls substituierte Aryl-($C_1$ bis $C_4$)-alkylgruppe, mit der Maßgabe dass, wenn $G^{25}$ für ein Wasserstoffatom steht, $G^{27}$ neben den vorgenannten Gruppen zusätzlich für eine Gruppe $-NH_2$ stehen kann,
- $G^{26}$ steht für ein Wasserstoffatom, eine ($C_1$ bis $C_4$)-Alkylgruppe, eine ($C_1$ bis $C_4$)-Monohydroxyalkylgruppe oder eine ($C_2$ bis $C_4$)-Polyhydroxyalkylgruppe und
- $G^{27}$ steht für ein Wasserstoffatom, eine gegebenenfalls substituierte Arylgruppe, eine ($C_1$ bis $C_4$)-Alkylgruppe oder eine ($C_1$ bis $C_4$)-Monohydroxyalkylgruppe, insbesondere für ein Wasserstoffatom oder eine Methylgruppe.

Bevorzugt bindet in Formel (E5) der Rest $-NG^{25}G^{26}$ an die 5 Position und der Rest $G^{27}$ an die 3 Position des Pyrazolzyklus.

Besonders bevorzugte Pyrazol-Derivate sind insbesondere die Verbindungen, die ausgewählt werden aus 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methyl-pyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Im folgenden werden Beispiele für die als Substituenten der Verbindungen der Formeln (E1) bis (E5) genannten Reste aufgezählt: Beispiele für ($C_1$ bis $C_4$)-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$. Erfindungsgemäße Beispiele für ($C_1$ bis $C_4$)-Alkoxyreste sind $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $-OC(CH_3)_3$, insbesondere eine Methoxy- oder eine Ethoxygruppe.

Weiterhin können als bevorzugte Beispiele für eine ($C_1$ bis $C_4$)-Monohydroxyalkylgruppe $-CH_2OH$, $-CH_2CH_2OH$, $-CH_2CH_2CH_2OH$, $-CHCH(OH)CH_3$, $-CH_2CH_2CH_2CH_2OH$, wobei die Gruppe $-CH_2CH_2OH$ bevorzugt ist. Ein besonders bevorzugtes Beispiel einer ($C_2$ bis $C_4$)-Polyhydroxyalkylgruppe ist die 1,2-Dihydroxyethylgruppe. Beispiele für Halogenatome sind F-, Cl- oder Br-Atome, Cl-Atome sind ganz besonders bevorzugte Beispiele. Beispiele für stickstoffhaltige Gruppen sind insbesondere $-NH_2$, ($C_1$ bis $C_4$)-Monoalkylaminogruppen, ($C_1$ bis $C_4$)-Dialkylaminogruppen, ($C_1$ bis $C_4$)-Trialkylammoniumgruppen, ($C_1$ bis $C_4$)-Monohydroxyalkylaminogruppen, Imidazolinium und $-NH_3^+$. Beispiele für ($C_1$ bis $C_4$)-Monoalkylaminogruppen sind $-NHCH_3$, $-NHCH_2CH_3$, $-NHCH_2CH_2CH_3$, $-NHCH(CH_3)_2$.

Beispiele für ($C_1$ bis $C_4$)-Dialkylaminogruppe sind $-N(CH_3)_2$, $-N(CH_2CH_3)_2$. Beispiele für ($C_1$ bis $C_4$)-Trialkylammoniumgruppen sind $-N^+(CH_3)_3$, $-N^+(CH_3)_2(CH_2CH_3)$, $-N^+(CH_3)(CH_2CH_3)_2$.

Beispiele für ($C_1$ bis $C_4$)-Hydroxyalkylaminoreste sind $-NH-CH_2CH_2OH$, $-NH-CH_2CH_2OH$, $-NH-CH_2CH_2CH_2OH$, $-NH-CH_2CH_2CH_2OH$. Beispiele für ($C_1$ bis $C_4$)-Alkoxy-($C_1$ bis $C_4$)-alkylgruppen sind die Gruppen $-CH_2CH_2-O-CH_3$, $-CH_2CH_2CH_2-O-CH_3$, $-CH_2CH_2-O-CH_2CH_3$, $-CH_2CH_2CH_2-O-CH_2CH_3$, $-CH_2CH_2-O-CH(CH_3)$, $-CH_2CH_2CH_2-O-CH(CH_3)$.

Beispiele für Hydroxy-($C_1$ bis $C_4$)-alkoxyreste sind $-O-CH_2OH$, $-O-CH_2CH_2OH$, $-O-CH_2CH_2CH_2OH$, $-O-CHCH(OH)CH_3$, $-O-CH_2CH_2CH_2CH_2OH$. Beispiele für ($C_1$ bis $C_4$)-Acetylaminoalkoxyreste sind $-O-CH_2NHC(O)CH_3$, $-O-CH_2CH_2NHC(O)CH_3$, $-O-CH_2CH_2CH_2NHC(O)CH_3$, $-O-CH_2CH(NHC(O)CH_3)CH_3$, $-O-CH_2CH_2CH_2CH_2NHC(O)CH_3$. Beispiele für ($C_1$ bis $C_4$)-Carbamoylaminoalkoxyreste sind $-O-CH_2CH_2-NH-C(O)-NH_2$, $-O-CH_2CH_2CH_2-NH-C(O)-NH_2$,

$-O-CH_2CH_2CH_2CH_2-NH-C(O)-NH_2$. Beispiele für ($C_1$ bis $C_4$)-Aminoalkylreste sind $-CH_2NH_2$, $-CH_2CH_2NH_2$, $-CH_2CH_2CH_2NH_2$, $-CH_2CH(NH_2)CH_3$, $-CH_2CH_2CH_2CH_2NH_2$.

Beispiele für ($C_1$ bis $C_4$)-Cyanoalkylreste sind $-CH_2CN$, $-CH_2CH_2CN$, $-CH_2CH_2CH_2CN$. Beispiele für ($C_1$ bis $C_4$)-Hydroxyalkylamino-($C_1$ bis $C_4$)-alkylreste sind $-CH_2CH_2NH-CH_2CH_2OH$, $-CH_2CH_2CH_2NH-CH_2CH_2OH$, $-CH_2CH_2NH-CH_2CH_2CH_2OH$, $-CH_2CH_2CH_2NH-CH_2CH_2CH_2OH$.

Beispiele für Di[($C_1$ bis $C_4$)-Hydroxyalkyl]amino-($C_1$ bis $C_4$)-alkylreste sind $-CH_2CH_2N(CH_2CH_2OH)_2$, $-CH_2CH_2CH_2N(CH_2CH_2OH)_2$, $-CH_2CH_2N(CH_2CH_2CH_2OH)_2$, $-CH_2CH_2CH_2N(CH_2CH_2CH_2OH)_2$. Ein Beispiel für Arylgruppen ist die Phenylgruppe. Beispiele für Aryl-($C_1$ bis $C_4$)-alkylgruppen sind die Benzylgruppe und die 2-Phenylethylgruppe.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:

- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1),

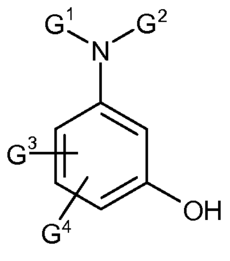

(K1)

worin

G¹ und G² unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_2$ bis $C_4$)-Perfluoracylgruppe, eine Aryl-($C_1$ bis $C_6$)-alkylgruppe, eine Amino-($C_1$ bis $C_6$)-alkylgruppe, eine ($C_1$ bis $C_6$)-Dialkylamino-($C_1$ bis $C_6$)-alkylgruppe oder eine ($C_1$ bis $C_6$)-Alkoxy-($C_1$ bis $C_6$)-alkylgruppe, wobei $G^1$ und $G^2$ gemeinsam mit dem Stickstoffatom einen fünfgliedrigen, sechsgliedrigen oder siebengliedrigen Ring bilden können,

G³ und G⁴ unabhängig voneinander stehen für ein Wasserstoffatom, ein Halogenatom, eine ($C_1$ bis $C_4$)-Alkylgruppe, eine ($C_1$ bis $C_4$)-Alkoxygruppe, eine Hydroxygruppe, eine ($C_1$ bis $C_4$)-Monohydroxyalkylgruppe, eine ($C_2$

bis $C_4$)-Polyhydroxyalkylgruppe, eine Hydroxy-($C_1$ bis $C_4$)-alkoxygruppe, eine ($C_1$ bis $C_6$)-Alkyox-($C_2$ bis $C_6$)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2),

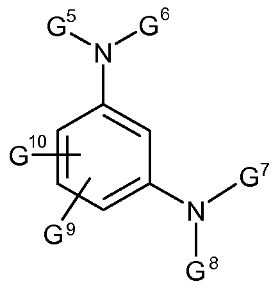

(K2)

worin

$G^5$, $G^6$, $G^7$ und $G^8$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-($C_1$ bis $C_4$)-alkylgruppe, eine ($C_2$ bis $C_4$)-Perfluoracylgruppe, oder gemeinsam mit dem Stickstoffatom einen fünfgliedrigen oder sechsgliedrigen Heterozyklus bilden

$G^9$ und $G^{10}$ unabhängig voneinander stehen für ein Wasserstoffatom, ein Halogenatom, eine ($C_1$ bis $C_4$)-Alkylgruppe, eine ω-(2,4-Diaminophenyl)-($C_1$ bis $C_4$)-alkylgruppe, eine ω-(2,4-Diaminophenyloxy)-($C_1$ bis $C_4$)-alkoxygruppe, eine ($C_1$ bis $C_4$)-Alkoxygruppe, eine Hydroxygruppe, eine ($C_1$ bis $C_4$)-Alkoxy-($C_2$ bis $C_4$)-alkoxygruppe, eine Arylgruppe, eine Heteroarylgruppe, eine ($C_1$ bis $C_4$)-Monohydroxyalkylgruppe, eine ($C_2$ bis $C_4$)-Polyhydroxyalkylgruppe, eine Hydroxy-($C_1$ bis $C_4$)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3),

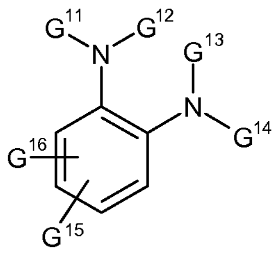

(K3)

worin

$G^{11}$, $G^{12}$, $G^{13}$ und $G^{14}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-($C_1$ bis $C_4$)-alkylgruppe, eine ($C_2$ bis $C_4$)-Perfluoracylgruppe, oder gemeinsam mit dem Stickstoffatom einen fünfgliedrigen oder sechsgliedrigen Heterozyklus bilden

$G^{15}$ und $G^{16}$ unabhängig voneinander stehen für ein Wasserstoffatom, ein Halogenatom, eine Carboxylgruppe, eine ($C_1$ bis $C_4$)-Alkylgruppe, eine ($C_1$ bis $C_4$)-Alkoxygruppe, eine Hydroxygruppe, eine ($C_1$ bis $C_4$)-Monohydroxyalkylgruppe, eine ($C_2$ bis $C_4$)-Polyhydroxyalkylgruppe, eine Hydroxy-($C_1$ bis $C_4$)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4),

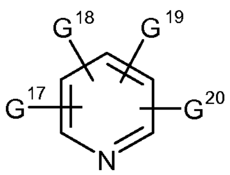

(K4)

worin

$G^{17}$ und $G^{18}$ stehen unabhängig voneinander für eine Hydroxygruppe oder eine Gruppe-$NG^{21}G^{22}$, worin $G^{21}$ und $G^{22}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine Arylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-($C_1$ bis $C_4$)-alkylgruppe,

$G^{19}$ und $G^{20}$ stehen unabhängig voneinander für ein Wasserstoffatom, ein Halogenatom, eine ($C_1$ bis $C_4$)-Alkylgruppe oder eine ($C_1$ bis $C_4$)-Alkoxygruppe.

Es ist bevorzugt, wenn gemäß Formel (K4) die Reste $G^{17}$ und $G^{18}$ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der

vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5),

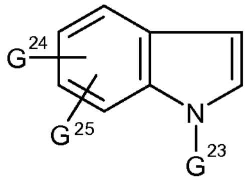

(K5)

worin

$G^{23}$ steht für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine Aryl-($C_1$ bis $C_4$)-alkylgruppe,

$G^{24}$ steht für eine Hydroxygruppe oder eine Gruppe -$NG^{26}G^{27}$, worin $G^{26}$ und $G^{27}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe,

$G^{25}$ Wasserstoffatom, ein Halogenatom oder eine ($C_1$ bis $C_4$)-Alkylgruppe,

mit der Maßgabe, dass $G^{24}$ in meta-Position oder ortho-Position zum Strukturfragment $NG^{23}$ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6),

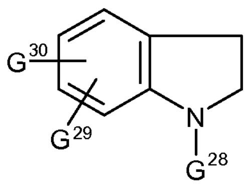

(K6)

worin

$G^{28}$ steht für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine Aryl-($C_1$ bis $C_4$)-alkylgruppe,

$G^{29}$ steht für eine Hydroxygruppe oder eine Gruppe -$NG^{31}G^{32}$, worin $G^{31}$ und $G^{32}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe,

$G^{30}$ Wasserstoffatom, ein Halogenatom oder eine ($C_1$ bis $C_4$)-Alkylgruppe,

mit der Maßgabe, dass $G^{29}$ in meta-Position oder ortho-Position zum Strukturfragment $NG^{28}$ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet

wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für ($C_1$ bis $C_4$)-Alkylreste sind die Gruppen $-CH_3$,$-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$.

Erfindungsgemäße Beispiele für ($C_3$ bis $C_6$)-Cycloalkylgruppen sind die Cyclopropyl, die Cyclopentyl und die Cyclohexylgruppe. Erfindungsgemäße Beispiele für ($C_1$ bis $C_4$)-Alkoxyreste sind $-OCH_3$,$-OCH_2CH_3$, $-OCH_2CH_2CH_3$, $-OCH(CH_3)_2$, $-OCH_2CH_2CH_2CH_3$, $-OCH2CH(CH_3)_2$, $-OCH(CH_3)CH_2CH_3$, $-OC(CH_3)_3$, insbesondere eine Methoxy- oder eine Ethoxygruppe.

Weiterhin können als bevorzugte Beispiele für eine ($C_1$ bis $C_4$)-Monohydroxyalkylgruppe $-CH_2OH$,$-CH_2CH_2OH$, $-CH_2CH_2CH_2OH$, $-CH_2CH(OH)CH_3$, $-CH_2CH_2CH_2CH_2OH$ genannt werden, wobei die Gruppe $-CH_2CH_2OH$ bevorzugt ist.

Ein besonders bevorzugtes Beispiel einer ($C_2$ bis $C_4$)-Polyhydroxyalkylgruppe ist die 1,2-Dihydroxyethylgruppe. Beispiele für Halogenatome sind F-, Cl- oder Br-Atome, Cl-Atome sind ganz besonders bevorzugte Beispiele. Beispiele für stickstoffhaltige Gruppen sind insbesondere $-NH_2$, ($C_1$ bis $C_4$)-Monoalkylaminogruppen, ($C_1$ bis $C_4$)-Dialkylaminogruppen, ($C_1$ bis $C_4$)-Trialkylammoniumgruppen, ($C_1$ bis $C_4$)-Monohydroxyalkylaminogruppen, Imidazolinium und $-NH_3^+$. Beispiele für ($C_1$ bis $C_4$)-Monoalkylaminogruppen sind $-NHCH_3$, $-NHCH_2CH_3$, $-NHCH_2CH_2CH_3$,-NHCH(CH3)2. Beispiele für ($C_1$ bis $C_4$)-Dialkylaminogruppe sind $-N(CH_3)_2$, $-N(CH_2CH_3)_2$. Beispiele für ($C_1$ bis $C_4$)-Alkoxy-($C_1$ bis $C_4$)-alkylgruppen sind die Gruppen $-CH_2CH_2-O-CH_3$, $-CH_2CH_2CH_2-O-CH_3$,$-CH_2CH_2-O-CH_2CH_3$, $-CH_2CH_2CH_2-O-CH_2CH_3$, $-CH_2CH_2-O-CH(CH_3)_2$, $-CH_2CH_2CH_2-O-CH(CH_3)_2$.

Beispiele für ($C_1$ bis $C_4$)-Alkoxy-($C_1$ bis $C_4$)-alkoxygruppen sind die Gruppen $-O-CH_2CH_2-O-CH_3$, $-O-CH_2CH_2CH_2-O-CH_3$, $-O-CH_2CH_2-O-CH_2CH_3$, $-O-CH_2CH_2CH_2-O-CH_2CH_3$, $-O-CH_2CH_2-O-CH(CH_3)_2$,$-O-CH_2CH_2CH_2-O-CH(CH_3)_2$.

Beispiele für Hydroxy-($C_1$ bis $C_4$)-alkoxyreste sind $-O-CH_2OH$, $-O-CH_2CH_2OH$, $-O-CH_2CH_2CH_2OH$, $-O-CH_2CH(OH)CH_3$, $-O-CH_2CH_2CH_2CH_2OH$.

Beispiele für ($C_1$ bis $C_4$)-Aminoalkylreste sind $-CH_2NH_2$, $-CH_2CH_2NH_2$, $-CH_2CH_2CH_2NH_2$, $-CH_2CH(NH_2)CH_3$, $-CH_2CH_2CH_2CH_2NH_2$. Ein Beispiel für Arylgruppen ist die Phenylgruppe, die auch substituiert sein kann. Beispiele für Aryl-($C_1$ bis $C_4$)-alkylgruppen sind die Benzylgruppe und die 2-Phenylethylgruppe.

Die erfindungsgemäßen Mittel enthalten bevorzugt als farbgebende Verbindung mindestens eine der folgenden Kombinationen a) bis d) von Oxidationsfarbstoffvorprodukten:

a) mindestens einen heterozyklischen Entwickler ausgewählt aus Pyrazolderivaten (insbesondere 1-(2-hydroxyethyl)pyrazol) und Pyrimidinderivaten (insbesondere 2,4,5,6-Tetrahydroxypyrimidon), mindestens eine Verbindung ausgewählt aus m-Aminophenol oder seiner Derivate als Kuppler,
b) 4-Amino-3-methylphenol, 5-Amino-2-methylphenol,
c) p-Toluylendiamin, 4-Amino-3-methylphenol, 5-Amino-2-methylphenol,
d) 2-(β-Hydroxyethyl)-p-phenylendiamin, 4-Amino-3-methylphenol, 5-Amino-2-methylphenol.

Die erfindungsgemäßen Mittel können zusätzlich mindestens einen direktziehenden Farbstoff enthalten. Dabei handelt sich um Farbstoffe, die direkt auf das Haar aufziehen und keinen oxidativen Prozess zur

Ausbildung der Farbe benötigen. Bevorzugte direktziehende Farbstoffe sind Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können ferner in anionische, kationische und nichtionische direktziehende Farbstoffe unterteilt werden.

Anionische direktziehende Farbstoffe: Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4- Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6- Hydroxy-5-[(4-sulfonaphth-1-yl)azo]-2,4-naphthalindisulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5- (Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'- dihydroxyspiro[isobenzofuran-1(3H),9'-[9H]xanthen]-3-on-dinatriumsalz (C.I. 45,380; Acid Red 87), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3',6'-Dihydroxy-4',5'-diiodospiro[isobenzofuran-1(3H),9'(9H)- xanthen]-3-on-dinatriumsalz (C.I. 45425; Acid Red 95), 2-Hydroxy-3-((2- hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195), 3-Hydroxy-4-[(4-methyl-2-sulfonphenyl)azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4- Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1H-indol-5-sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2- sulfophenyl)amino]-9,10-anthrachinon-natriumsalz (C.I. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau), 3,4,5,6,3',3",5',5"-Octabromphenolsulfonphthalein (Tetrabromphenolblau). Bevorzugte anionische direktziehende Farbstoffe sind die unter den internationalen Bezeichnungen bzw. Handelsnamen Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten

Verbindungen.

Kationische direktziehende Farbstoffe: Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I. 42,595; Basic Blue 7), Di-(4-(dimethylamino)phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(dimethylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1(4H)-naphthalinon-chlorid (C.I. 56,059; Basic Blue No. 99), Bis[4-(dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5-dimethoxyphenyl)azo]-N,N,N-trimethylbenzolaminiumchlorid (C.I. 12,605, Basic Orange 69), 3,7-Diamino-2,8-dimethyl- 5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4-(dimethylamino)phenyl)-phenylmethanol (C.I. 42,000; Basic Green 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehenden Farbstoffe sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

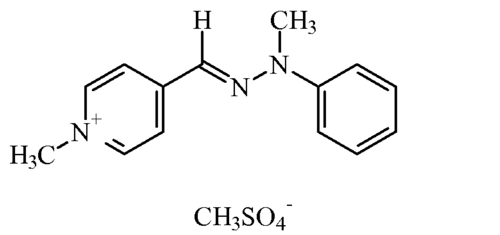

(DZ1)

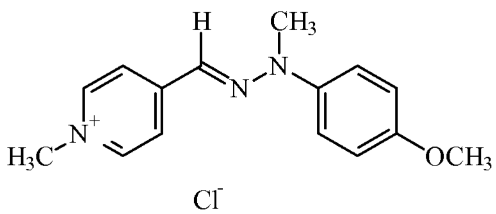

(DZ2)

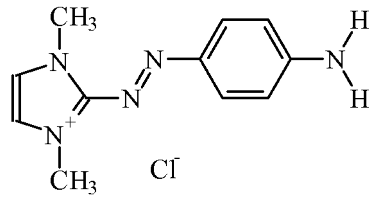

(DZ3)

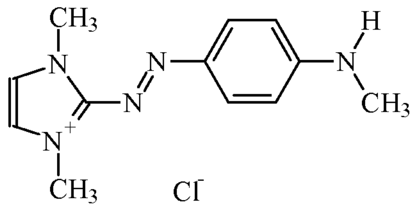

(DZ4)

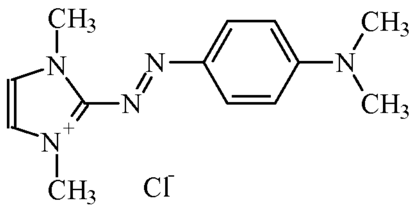

(DZ5)

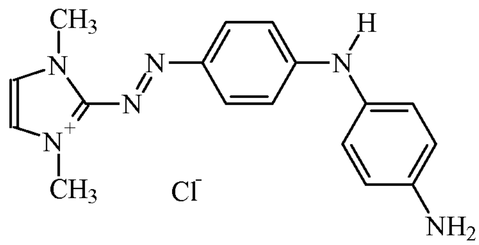

(DZ6)

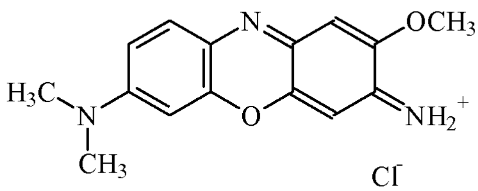

(DZ7)

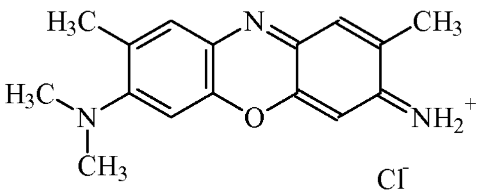

(DZ8)

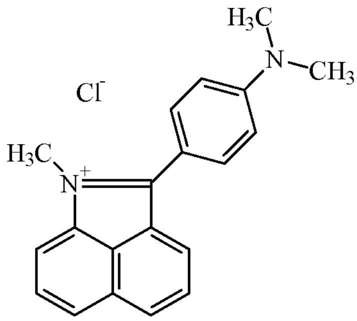

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Nichtionische direktziehende Farbstoffe: Als nichtionische direktziehende Farbstoffe eignen sich insbesondere nichtionische Nitro- und Chinonfarbstoffe und neutrale Azofarbstoffe.

Geeignete neutrale Azofarbstoffe sind insbesondere: 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Als farbgebende Verbindungen der Vorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins der Formel (RN1),

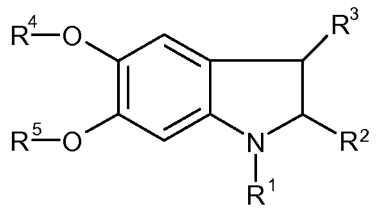

(RN1)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (RN2),

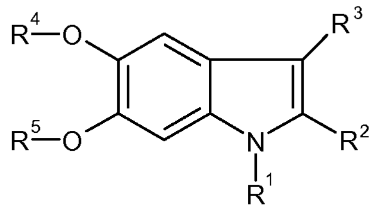

(RN2)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- beziehungsweise die Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Die erfindungsgemäß verwendeten Sternpolymere entfalten ihre Wirkung besonders in Mitteln zur oxidativen Färbung keratinhaltiger Fasern, insbesondere menschlicher Haare. Es hat sich daher als bevorzugt herausgestellt, ein erfindungsgemäßes Mittel zur oxidativen Färbung keratinhaltiger Fasern zu verwenden, umfassend in einem kosmetischen Träger

(a) mindestens einem Sternpolymer, umfassend ein als Zentrum fungierendes Strukturfragment und mindestens drei radial an dieses Zentrum gebundene Gruppen, wobei diese Gruppen

(i) jeweils mindestens eine Polyetherstruktureinheit aufweisen, die eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) mindestens einen Rest aufweisen, enthaltend

- mindestens eine Gruppe *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht, und/oder
- mindestens eine anionische Gruppe und/oder
- mindestens ein quaternisiertes Stickstoffatom,

und
(b) mindestens Oxidationsfarbstoffvorprodukt vom Typ der Entwicklerkomponenten und gegebenenfalls zusätzlich mindestens eine Kupplerkomponente als farbgebende Verbindung,
und
(c) mindestens ein Oxidationsmittel.

Für die Komponenten (a) und (b) des erfindungsgemäßen Mittels dieser Ausführungsform gelten jeweils die zuvor genannten bevorzugten Merkmale (*vide supra*).

Die Oxidationsmittel im Sinne der Erfindung sind von Luftsauerstoff verschieden und besitzen ein solches Oxidationspotenzial, das es ermöglicht ein Oxidationsfarbstoffvorprodukt vom Entwicklertyp zu oxidieren. Als Oxidationsmittel kommt bevorzugt Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n $H_2O_2$ (n ist eine positive ganze Zahl größer 0), Harnstoffperoxid und Melaminperoxid, in Frage.

Das Oxidationsmittel ist bevorzugt in einer Menge von 1,0 bis 10 Gew.-%, insbesondere von 3,0 bis 10,0 Gew.-%, jeweils bezogen auf das Gewicht des anwendungsbereiten kosmetischen Färbemittels, in dem kosmetischen Mittel enthalten.

In einer bevorzugten Ausführungsform der Erfindung wird das erfindungsgemäße kosmetische Färbemittel vor der Applikation aus einer ersten Zusammensetzung, enthaltend in einem kosmetischen Träger mindestens eine farbverändernde Komponente, und einer zweiten Zusammensetzung, enthaltend in einem kosmetischen Träger mindestens ein Oxidationsmittel, gemischt, mit der Massgabe, dass die erste Zusammensetzung oder/und die zweite Zusammensetzung mindestens ein wie im zweiten Erfindungsgegenstand definiertes Sternpolymer enthält. Die erste und zweite Zusammensetzung werden getrennt voneinander in jeweils einem Kompartiment konfektioniert und gemeinsam in einer Verpackungseinheit (Kit) bereitgestellt. Kurz vor der Anwendung werden beide Komponenten miteinander vermischt. Das dabei entstehende gebrauchsfertige Färbepräparat weist bevorzugt einen pH-Wert im Bereich von 1 bis 12, besonders bevorzugt von pH 1 bis 10, auf.

Das erfindungsgemäße kosmetische Färbemittel enthält bevorzugt mindestens ein Alkalisierungsmittel. Die erfindungsgemäß verwendbaren Alkalisierungsmittel und werden bevorzugt ausgewählt aus mindestens einem Vertreter der Gruppe, die gebildet wird, aus Ammoniak, basischen Aminosäuren, Alkalihydroxiden, Alkanolaminen, Alkalimetallmetasilikaten, Harnstoff, Alkaliphosphaten und Alkalihydrogenphosphaten. Als Alkalimetallionen dienen bevorzugt Lithium, Natrium, Kalium, insbesondere Natrium oder Kalium. Wiederum bevorzugt sind die Alkalisierungsmittel von Ammoniak verschieden.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, L-Arginin, D-Arginin, DL-Arginin, N-Methylglucamin und Harnstoff.

Ferner können die erfindungsgemäßen Färbemittel weitere Wirk-, Hilfs- und Zusatzstoffe, wie beispielsweise nichtionische Polymere; kationische Polymere; zwitterionische und amphotere Polymere; anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/Malein-säureanhydrid-Copolymere und Acrylsäure/Ethylacrylat/N-tert.Butyl-acrylamid-Terpolymere; haarkonditionierende Verbindungen wie Phospholipide; Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; kationische Tenside; Entschäumer; Antischuppenwirkstoffe ; Lichtschutzmittel, insbesondere derivatisierte Benzophenone, Zimtsäure-Derivate und Triazine; Wirkstoffe wie Allantoin, Pyrrolidoncarbonsäuren und deren Salze sowie Bisabolol; Vitamine, Provitamine und Vitaminvorstufen, insbesondere solche der Gruppen A, $B_3$, $B_5$, $B_6$, C, E, F und H; Pflanzenextrakte insbesondere die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng oder Ingwerwurzel; Cholesterin; Konsistenzgeber; Fette und Wachse; Komplexbildner; Quell- und Penetrationsstoffe; Trübungsmittel; Perlglanzmittel; festförmige Pigmente; Stabilisierungsmittel für Wasserstoffperoxid und andere Oxidationsmittel; Treibmittel; Antioxidantien; enthalten.

Die bevorzugten Ausführungsformen des ersten Erfindungsgegenstandes gelten für den zweiten Erfindungsgegenstand *mutatis mutandis.*

Ein dritter Gegenstand der vorliegenden Erfindung ist ein kosmetisches Verfahren zur Färbung keratinhaltiger Fasern, bei dem ein Mittel des zweiten Erfindungsgegenstandes auf die keratinhaltigen Fasern (insbesondere menschliche Haare) aufgetragen wird und nach einer Einwirkungszeit wieder abgespült wird.

Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die bevorzugten Ausführungsformen des ersten und zweiten Erfindungsgegenstandes gelten für den dritten Erfindungsgegenstand *mutatis mutandis.*

## Beispiele

Die im Beispielteil angegebenen Molekulargewichte sind zahlenmittlere Molekulargewichte der Ausgangsverbindungen (Polyether-Polyole), die zur Herstellung der Präpolymere eingesetzt wurden. Das zahlenmittlere Molekulargewicht der Polyole lässt sich durch Endgruppenbestimmung rechnerisch aufgrund der Kenntnis der Funktionalität der Verbindungen oder der Funktionalität der Mischungskomponenten und der OH-Zahl der Verbindung oder der Mischung (ermittelt nach DIN 53240) bestimmen. Für den Fall, dass als Ausgangsverbindungen andere Verbindungen anstelle der Polyole verwendet werden, ist deren zahlenmittleres Molekulargewicht maßgeblich. So kann beispielsweise das zahlenmittlere Molekulargewicht von Aminen über eine Endgruppenbestimmung durch potentiometrische Titration nach der DIN 16945 ermittelt werden.

Herstellung geeigneter wasserlöslicher Polymere:

<u>Beispiel 1: Dreiarmiger Triethoxysilyl-terminierter Polvether (PP1):</u>

Das verwendete Polyether-Polyol ist ein 3-armiges statistisches Poly(ethylenoxid-co-propylenoxid) mit einem EO/PO-Verhältnis von 75/25 und mit einem zahlenmittleren Molekulargewicht von ca. 5000 g/mol, das von der Firma DOW Chemicals bezogen wurde (Voranol® CP 1421). Vor der Umsetzung wurde das Polyol im Vakuum unter Rühren für 1 h auf 80 °C erwärmt.

Zum getrockneten Polyether-Polyol (2,04 g, 0,41 mmol) wurde (3-Isocyanatopropyl)triethoxysilan (317 mg, 1,0 eq.) langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme des sternförmigen Präpolymers vorhanden

ist. Das Produkt ist eine farblose, viskose Flüssigkeit.

Beispiel 2: Sechsarmiger Triethoxysilyl-terminierter Polvether (PP2):

Das verwendete Polyether-Polyol ist ein 6-armiges statistisches Poly(ethylenoxid-co-propylenoxid) mit einem EO/PO-Verhältnis von 80/20 und mit einem Molekulargewicht von 12000 g/mol, das durch anionische ringöffnende Polymerisation von Ethylenoxid und Propylenoxid unter Verwendung von Sorbitol als Initiator hergestellt wurde. Vor der Umsetzung wurde das Polyol im Vakuum unter Rühren für 1 h auf 80 °C erwärmt.

Eine Lösung von Polyether-Polyol (3 g, 0,25 mmol), Triethylendiamin (9 mg, 0,081 mmol) und Dibutylzinndilaureat (9 mg, 0,014 mmol) in 25 ml wasserfreiem Toluol wurde vorgelegt, dazu wurde eine Lösung von (3-Isocyanatopropyl)triethoxysilan (0,6 ml, 2,30 mmol) in 10 ml wasserfreiem Toluol tropfenweise zugegeben. Die Lösung wurde weiter bei 50°C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches jeweils eine Triethoxylsilyl-Gruppe an den freien Enden der Polymerarme des sternförmigen Präpolymers aufweist, als eine farblos viskose Flüssigkeit. IR (Film, $cm^{-1}$): 3349 (m, -CO-NH-), 2868 (s, $-CH_2-$,$-CH_3$), 1719 (s, -C=O), 1456 (m, $-CH_2-$, $-CH_3$), 1107 (s, -C-O-C-), 954 (m, -Si-O-). $^1H$-NMR (Benzol-$d_6$, ppm): 1,13 (d, $-CH_3$ von Polymerarmen), 1,21 (t, $-CH_3$ von Silan-Endgruppen), 3,47 (s, $-CH_2$ von Polymerarmen), 3,74 (q, $-CH_2$ von Silan-Endgruppen).

Beispiel 3: Mischung aus dreiarmigem Triethoxysilyl-terminiertem Polvether und achtarmigem Triethoxvsilvl-terminiertem Polvether (PP3):

Die verwendete Polyether-Polyol-Mischung besteht aus einem 3-armigen statistischen Poly(ethylenoxid-co-propylenoxid) (Glycerin-gestartet) und einem 8-armigen Polyether-Polyol (Rohrzucker-gestartet). Die Polymerarme sind jeweils statistische Poly(ethylenoxid-co-propylenoxide) mit einem EO/PO-Verhältnis von 75/25. Die OH-Funktionalität beträgt durchschnittlich 6,9 (durch Endgruppenbestimmung ermittelt), wodurch sich ein durchschnittliches zahlenmittleres Molekulargewicht von ca. 12000 g/mol ergibt. Es ergibt sich ein Verhältnis von 78 Gew.-% an 8-armigem Polyether-Polyol zu 22 Gew.-% an 3-armigem Polyether-Polyol. Das Polyether-Polyol-Gemisch wurde von der Firma DOW Chemicals bezogen (Voranol® 4053). Vor der Umsetzung wurde das Polyether-Polyol im Vakuum unter Rühren für 1 h auf 80 °C erwärmt.

Zum getrockneten Polyether-Polyol (209 g, 16,9 mmol) wurde Dibutylzinndilaureat (20,9 mg, 0,01 %) und (3-Isocyanatopropyl)triethoxysilan (30,3 g, 1,0 eq.) langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme der beiden sternförmigen Präpolymere vorhanden ist. Das Produkt ist eine farblose, viskose Flüssigkeit.

2.0 Färbung

In die Färbecreme des Handelsprodukts Igora Royal 6-88 (Schwarzkopf) wurden bezogen auf das Gesamtgewicht der Mischung 1,0 Gew.-% eines der wasserlöslichen Polymere PP1 bzw. PP2 eingearbeitet. Die resultierende modifizierte Färbecreme wurde ummittelbar vor der Applikation auf Haarsträhnen (1 g standardisiertes Haar "European natural hair 6/0" Charge # 06/2010, N93 der Firma Kerling International, Deutschland, an einem Ende zum Haarbündel verklebt) mit einer handelsüblichen 6 Gew.-% Wasserstoffperoxid-haltigen Entwicklerdispersion Oxigenta (Schwarzkopf) im Gewichtsverhältnis 1 zu 1 zu einem erfindungsgemäßen Haarfärbemittel gemischt.

Das anwendungsbereite Färbemittel wurde danach im Gewichtsverhältnis 4 g Färbemittel auf 1 g Haar auf eine Haarsträhne aufgetragen, 30 Minuten bei 32°C einwirken gelassen und von der Faser gespült. Es wurden insgesamt 4 Haarsträhnen damit gefärbt. Darüber hinaus wurden 4 Haarsträhnen nach obiger Vorgehensweise mit dem obigen Handelsprodukt ohne Zusatz des besagten Sternpolymers (nicht erfindungsgemäß) eingefärbt.

Die Strähnen wurde jeweils trocknen gelassen und farbmetrisch unter Bestimmung der L, a, b Ausgangswerte je Strähne gemessen (Gerät Spectraflash 450, Software Colortools). Je Haarsträhne wurden 8 Messpunkte genommen und für jeden Wert der erfindungsgemäß gefärbten Strähnen jeweils sortiert nach Sternpolymer und der nichterfindungsgemäß gefärbten Strähnen das jeweilige arithmetische Mittel unter Erhalt der jeweiligen Werte $L_0$, $a_0$, $b_0$ bestimmt. Genauso wurde bei den folgenden farbmetrischen Messungen verfahren.

Um die Waschechtheit zu ermitteln, wurden die Haarsträhnen einer Waschprozedur unterzogen, die die Haarwäsche simuliert:

Ein Ultraschallbad wurde mit wässriger Shampoo-Lösung (2 Gew.-% Schaumaschampoo "7 Kräuter") gefüllt. Die colorierten Haarsträhnen wurden in diese Waschlösung getaucht und darin 15 min mit Ultraschall (Stufe 5) behandelt. Diese Behandlung entspricht der Reinigungsleistung von sechs Haarwäschen. Anschließend wurden die Strähnen gründlich gespült, getrocknet und erneut farbmetrisch gemessen. Die Strähnen wurden sodann einem weiteren Waschzyklus

unterworfen. Die Bewertung der Waschechtheit wurde über die Berechnung des Farbabstandes der Strähne vor dem Waschen und nach dem jeweiligen Waschzyklus vorgenommen und ermittelt sich aus:

$$\Delta E = [(L_i\text{-}L_0)^2 + (a_i - a_0)^2 + (b_i - b_0)^2]^{1/2}$$

$L_i$, $a_i$, $b_i$ Werte nach 6, 12, 18, 24 bzw. 36 Haarwäschen
$L_0$, $a_0$, $b_0$ Werte nach 0 Haarwäschen Haarsträhne

Tabelle 1: ΔE nach 6, 12, 18, 24 und 30 Wäschen

| Anzahl Wäschen | 6 | 12 | 18 | 24 | 30 |
|---|---|---|---|---|---|
| Handelprodukt erfindungsgemäßes | 2.8 | 3.8 | 4.6 | 5.9 | 6.8 |
| Mittel mit PP3 | 2.6 | 3.4 | 4.1 | 4.4 | 4.9 |

**Patentansprüche**

**1.** Verwendung von Sternpolymeren, umfassend ein als Zentrum fungierendes Strukturfragment und mindestens drei radial an dieses Zentrum gebundene Gruppen, wobei diese Gruppen

(i) jeweils mindestens eine Polyetherstruktureinheit aufweisen, die eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) mindestens einen Rest aufweisen, enthaltend

• mindestens eine Gruppe *-$Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht, und/oder
• mindestens eine anionische Gruppe und/oder
• mindestens ein quaternisiertes Stickstoffatom,

zur Verbesserung der Farbechtheit künstlicher Färbungen farbgebender Verbindungen auf keratinhaltigen Fasern, insbesondere menschlichen Haaren,
wobei die farbgebenden Verbindungen aus mindestens einem Oxidationsfarbstoffvorprodukt vom Typ der Entwicklerkomponenten und gegebenenfalls zusätzlich mindestens einer Kupplerkomponente ausgewählt werden.

**2.** Kosmetisches Mittel zur Färbung keratinhaltiger Fasern, insbesondere menschlicher Haare, enthaltend in einem kosmetischen Träger

(a) mindestens ein Sternpolymer, umfassend ein als Zentrum fungierendes Strukturfragment und mindestens drei radial an dieses Zentrum gebundene Gruppen, wobei diese Gruppen

(i) jeweils mindestens eine Polyetherstruktureinheit aufweisen, die eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) mindestens einen Rest aufweisen, enthaltend

• mindestens eine Gruppe *-$Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht, und/oder
• mindestens eine anionische Gruppe und/oder
• mindestens ein quaternisiertes Stickstoffatom,

und
(b) mindestens eine farbgebende Verbindung,

wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird.

3. Kosmetisches Mittel zur Färbung nach Anspruch 2, **dadurch gekennzeichnet, dass** das Sternpolymer aus mindestens einer Verbindung der allgemeinen Formel (PE-1) ausgewählt wird

$$[Q]\!-\!(K'\text{-}A\text{-}K\text{-}T)_n \qquad \text{(PE-1)}$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T steht für ein Strukturfragment, umfassend

- mindestens eine Gruppe $*\text{-Si(OR)}_x(\text{R'})_{3\text{-}x}$, worin R und R' unabhängig voneinander für eine ($C_1$ bis $C_4$)-Alkylgruppe (insbesondere für Methyl oder Ethyl) stehen, x für 1, 2 oder 3 steht, oder
- mindestens eine anionische Gruppe, oder
- mindestens ein quaternisiertes Stickstoffatom,

Q bedeutet ein als Zentrum fungierendes, organisches Strukturfragment, abgeleitet von linearen, verzweigten, zyklischen oder heterozyklischen Kohlenwasserstoffen, wobei all diese jeweils gesättigt, ungesättigt oder aromatisch sein dürfen,
n steht für eine ganze Zahl von 3 bis 64 (insbesondere für 3, 4, 5, 6, 7 oder 8).

4. Kosmetisches Mittel zur Färbung nach Anspruch einem der Ansprüche 2 oder 3, **dadurch gekennzeichnet, dass** das Sternpolymer als Zentrum einen Feststoffpartikel aufweist, welcher an der Oberfläche mit mindestens drei Resten *-K'-A-K-T modifiziert ist, worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T steht für ein Strukturfragment, umfassend

- mindestens eine Gruppe $*\text{-Si(OR)}_x(\text{R'})_{3\text{-}x}$, worin R und R' unabhängig voneinander für eine ($C_1$ bis $C_4$)-Alkylgruppe (insbesondere für Methyl oder Ethyl) stehen, x für 1, 2 oder 3 steht, oder
- mindestens eine anionische Gruppe, oder
- mindestens ein quaternisiertes Stickstoffatom.

5. Kosmetisches Mittel zur Färbung nach einem der Ansprüche 3 oder 4, **dadurch gekennzeichnet, dass** A steht für ein Strukturfragment der Formel (A1)

$$*\text{-}(OCH_2CH_2)_n\text{-}(OCH_2CH(CH_3))_m\text{-}* \qquad \text{(A1)}$$

worin

n eine ganze Zahl von 1 bis 500 bedeutet,
m eine ganze Zahl von 0 bis 500 bedeutet und
das Strukturfragment der Formel (A1) einen maximalen Anteil von 50 Gew.-% an Propylenoxid-Einheiten, bezogen auf das Gewicht des Strukturfragments (A1) hat.

6. Kosmetisches Mittel zur Färbung nach einem der Ansprüche 3 bis 5, **dadurch gekennzeichnet, dass** die Reste K und K' unabhängig voneinander für eine kovalente Bindung, eine Oxygruppe, eine Iminogruppe, eine ($C_1$ bis $C_6$)-Alkylengruppe oder mindestens eine der folgenden Konnektivitäten (K1) bis (K10) stehen

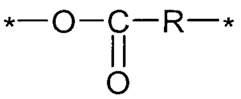 (K1)

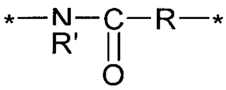 (K2)

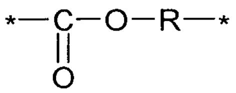 (K3)

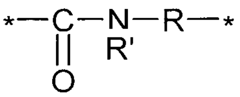 (K4)

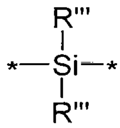 (K5)

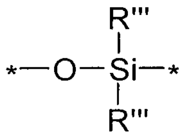 (K6)

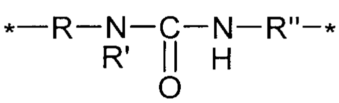 (K7)

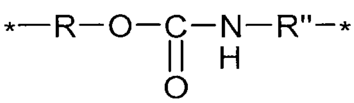 (K8)

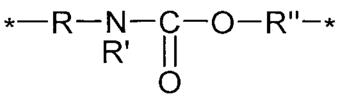 (K9)

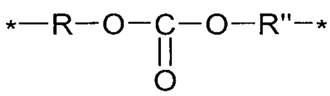 (K10)

worin

R und R'' unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,
R' ein Wasserstoffatom oder eine ($C_1$ bis $C_4$)-Alkylgruppe bedeutet,
R''' unabhängig voneinander eine ($C_1$ bis $C_4$)-Alkylgruppe oder eine Arylgruppe bedeutet.

**7.** Kosmetisches Mittel zur Färbung nach einem der Ansprüche 2 bis 6, **dadurch gekennzeichnet, dass** die besagten Sternpolymere in einer Menge von 0,01 bis 10,0 Gew.-%, bevorzugt von 0,1 bis 2,0 Gew.-%, besonders bevorzugt 0,2 bis 1,0 Gew.-%, jeweils bezogen auf das Gewicht des gesamten Mittels, enthalten sind.

**8.** Kosmetisches Mittel zur Färbung nach einem der Ansprüche 2 bis 7, **dadurch gekennzeichnet, dass** es zusätzlich mindestens eine Verbindung ausgewählt unter organischen Aminen umfassend 2 bis 20 Kohlenstoffatome, Carboxylatkomplexverbindungen des Zinns, Alkoxidverbindungen des Zinns, Carboxylatkomplexverbindungen des Bleis, Organoaluminiumverbindungen, Metallkomplexe von organischen Dicarbonylverbindungen, Metallkomplexe von organischen Dicarbonsäureestern, enthält.

**9.** Kosmetisches Mittel zur Färbung nach einem der Ansprüche 2 bis 8, **dadurch gekennzeichnet, dass** zusätzlich mindestens ein Oxidationsmittel, insbesondere Wasserstoffperoxyd und/oder mindestens ein Anlagerungsprodukt davon, enthalten ist.

**10.** Kosmetisches Verfahren zur Färbung keratinhaltiger Fasern, bei welchem ein kosmetisches Mittel nach einem der Ansprüche 2 bis 9 auf die keratinhaltigen Fasern (insbesondere menschliche Haare) aufgetragen und nach einer Einwirkungszeit wieder abgespült wird.

## Claims

1. The use of star polymers, comprising a structural fragment acting as a center and at least three groups radially bound to this center, wherein these groups

   (i) each have at least one polyether structural unit, which comprises a polyoxyalkylene chain consisting of ethylene oxide units or consisting of ethylene oxide units and propylene oxide units, having a maximum proportion of 50 wt.% of propylene oxide units, based on the weight of the polyoxyalkylene chain, and
   (ii) have at least one functional group, containing

   - at least one *-$Si(OR)_x(R')_{3-x}$group, where R and R' represent, independently of one another, a ($C_1$ to $C_4$) alkyl group (in particular methyl or ethyl), x represents 1, 2 or 3, and/or
   - at least one anionic group and/or
   - at least one quaternized nitrogen atom,

   for improving the color fastness of synthetic colorings of chromophoric compounds on keratin-containing fibers, in particular human hair,
   wherein the chromophoric compounds are selected from at least one oxidation dye precursor of the developer component type and optionally additionally at least one coupler component.

2. A cosmetic agent for coloring keratin-containing fibers, in particular human hair, containing in a cosmetic carrier

   (a) at least one star polymer, comprising a structural fragment acting as a center and at least three groups radially bound to this center, wherein these groups

   (i) each have at least one polyether structural unit, which comprises a polyoxyalkylene chain consisting of ethylene oxide units or consisting of ethylene oxide units and propylene oxide units, having a maximum proportion of 50 wt.% of propylene oxide units, based on the weight of the polyoxyalkylene chain, and
   (ii) have at least one functional group, containing

   - at least one *-$Si(OR)_x(R')_{3-x}$ group, where R and R' represent, independently of one another, a ($C_1$ to $C_4$) alkyl group (in particular methyl or ethyl), x represents 1, 2 or 3, and/or
   - at least one anionic group and/or
   - at least one quaternized nitrogen atom, and

   (b) at least one chromophoric compound,

   wherein the chromophoric compound is selected from at least one oxidation dye precursor of the developer component type and optionally additionally at least one coupler component.

3. The cosmetic agent for coloring according to claim 2, **characterized in that** the star polymer is selected from at least one compound of the general formula (PE-1)

$$[Q]\!-\!(K'\text{-}A\text{-}K\text{-}T)_n \qquad \text{(PE-1)}$$

   where

   A denotes a polyoxyalkylene chain consisting of ethylene oxide units or consisting of ethylene oxide units and propylene oxide units, having a maximum proportion of 50 wt.% of propylene oxide units, based on the weight of A,
   K and K' represent, independently of one another, a connectivity, selected from a covalent bond or from a molecular fragment having two free valencies,
   T represents a structural fragment, comprising

   - at least one *-$Si(OR)_x(R')_{3-x}$ group, where R and R' represent, independently of one another, a ($C_1$ to $C_4$) alkyl group (in particular methyl or ethyl), x represents 1, 2 or 3, or
   - at least one anionic group, or
   - at least one quaternized nitrogen atom,

Q denotes an organic structural fragment acting as a center, derived from linear, branched, cyclic or heterocyclic hydrocarbons, all of which hydrocarbons may each be saturated, unsaturated or aromatic,
n represents an integer from 3 to 64 (in particular 3, 4, 5, 6, 7 or 8).

**4.** The cosmetic agent for coloring according to one of claims 2 or 3, **characterized in that** the star polymer has a solid particle as a center, which is modified on the surface with at least three functional *-K'-A-K-T groups, where

A denotes a polyoxyalkylene chain consisting of ethylene oxide units or consisting of ethylene oxide units and propylene oxide units, having a maximum proportion of 50 wt.% of propylene oxide units, based on the weight of A,
K and K' represent, independently of one another, a connectivity, selected from a covalent bond or from a molecular fragment having two free valencies,
T represents a structural fragment, comprising

- at least one *-Si(OR)$_x$(R')$_{3-x}$ group, where R and R' represent, independently of one another, a ($C_1$ to $C_4$) alkyl group (in particular methyl or ethyl), x represents 1, 2 or 3, or
- at least one anionic group, or
- at least one quaternized nitrogen atom.

**5.** The cosmetic agent for coloring according to one of claims 3 or 4, **characterized in that** A represents a structural fragment of the formula (A1)

$$*\text{-}(OCH_2CH_2)_n\text{-}(OCH_2CH(CH_3))_m\text{-}* \qquad (A1)$$

where

n denotes an integer from 1 to 500,
m denotes an integer from 0 to 500, and
the structural fragment of formula (A1) has a maximum proportion of 50 wt.% of propylene oxide units, based on the weight of the structural fragment (A1).

**6.** The cosmetic agent for coloring according to one of claims 3 to 5, **characterized in that** the functional groups K and K' represent, independently of one another, a covalent bond, an oxy group, an imino group, a ($C_1$ to $C_6$) alkylene group or at least one of the following connectivities (K1) to (K10),

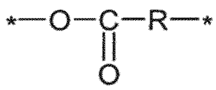
(K1)

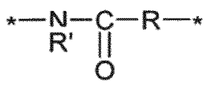
(K2)

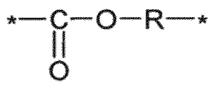
(K3)

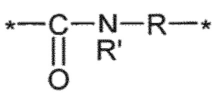
(K4)

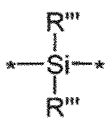
(K5)

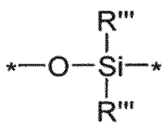
(K6)

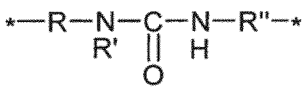
(K7)

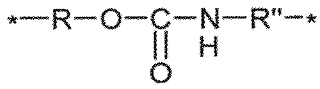
(K8)

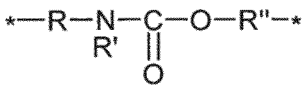
(K9)

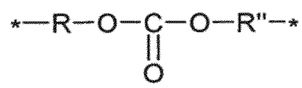
(K10)

where

R and R" represent, independently of one another, methylene, ethane-1,2-diyl, propane-1,2-diyl, propane-1,3-diyl, butane-1,2-diyl, butane-1,3-diyl, butane-1,4-diyl or phenylene,
R’ denotes a hydrogen atom or a ($C_1$ to $C_4$) alkyl group,
R'" denotes, independently of one another, a ($C_1$ to $C_4$) alkyl group or an aryl group.

7. The cosmetic agent for coloring according to one of claims 2 to 6, **characterized in that** said star polymers are contained in an amount of from 0.01 to 10.0 wt.%, preferably from 0.1 to 2.0 wt.%, particularly preferably from 0.2 to 1.0 wt.%, in each case based on the weight of the total agent.

8. The cosmetic agent for coloring according to one of claims 2 to 7, **characterized in that** it additionally contains at least one compound selected from organic amines comprising from 2 to 20 carbon atoms, carboxylate complex compounds of tin, alkoxide compounds of tin, carboxylate complex compounds of lead, organoaluminum compounds, metal complexes of organic dicarbonyl compounds and metal complexes of organic dicarboxylic acid esters.

9. The cosmetic agent for coloring according to one of claims 2 to 8, **characterized in that** at least one oxidizing agent, in particular hydrogen peroxide and/or at least one addition product thereof, is additionally contained.

10. A cosmetic method for coloring keratin-containing fibers, wherein a cosmetic agent according to one of claims 2 to 9 is applied to the keratin-containing fibers (in particular human hair) and is rinsed off again after a contact time.

**Revendications**

1. Utilisation de polymères en étoile, comprenant un fragment structurel faisant office de centre et au moins trois groupes liés à ce centre de manière radiale, dans laquelle ces groupes

(i) présentent respectivement au moins une unité structurelle de polyéther, qui comprennent une chaîne polyoxyalkylène à partir d’unités d’oxyde d’éthylène ou à partir d’unités d’oxyde d’éthylène et d’oxyde de propylène, avec une part maximale de 50 % en poids en unités d’oxyde de propylène, rapportée au poids de la chaîne polyoxyalkylène, et
(ii) présentent au moins un résidu contenant

• au moins un groupe $*-Si(OR)_x(R')_{3-x}$, dans lequel R et R’ représentent indépendamment l’un de l’autre un groupe alkyle ($C_1$ à $C_4$) (en particulier du méthyle ou de l’éthyle), x représente 1, 2 ou 3, et/ou
• au moins un groupe anionique et/ou
• au moins un atome d’azote quaternaire,

pour l’amélioration de l’authenticité de la couleur de composés colorants de colorations artificielles sur des fibres kératiniques, en particulier des cheveux humains,
dans laquelle les composés colorants sont sélectionnés parmi au moins un précurseur de colorant par oxydation du type des composants développeurs et le cas échéant en complément au moins un composant coupleur.

2. Agent cosmétique pour la coloration de fibres kératiniques, en particulier de cheveux humains, contenant dans un support cosmétique

(a) au moins un polymère en étoile, comprenant un fragment structurel faisant office de centre et au moins trois

groupes liés à ce centre de manière radiale, dans laquelle ces groupes

(i) présentent respectivement au moins une unité structurelle de polyéther, qui comprennent une chaîne polyoxyalkylène à partir d'unités d'oxyde d'éthylène ou à partir d'unités d'oxyde d'éthylène et d'oxyde de propylène, avec une part maximale de 50 % en poids en unités d'oxyde de propylène, rapportée au poids de la chaîne polyoxyalkylène, et
(ii) présentent au moins un résidu contenant

- au moins un groupe $*\text{-}Si(OR)_x(R')_{3-x}$, dans lequel R et R' représentent indépendamment l'un de l'autre un groupe alkyle ($C_1$ à $C_4$) (en particulier du méthyle ou de l'éthyle), x représente 1, 2 ou 3, et/ou
- au moins un groupe anionique et/ou
- au moins un atome d'azote quaternaire, et

(b) au moins un composé colorant,

dans laquelle le composé colorant est sélectionné parmi au moins un précurseur de colorant par oxydation du type des composants développeurs et le cas échéant en complément au moins un composant coupleur.

3. Agent cosmétique de coloration selon la revendication 2, **caractérisé en ce que** le polymère en étoile est sélectionné parmi au moins un composé de la formule générale (PE-1)

$$[Q]\text{—}(K'\text{-}A\text{-}K\text{-}T)_n \qquad \text{(PE-1)}$$

dans laquelle

A représente une chaîne polyoxyalkylène à partir d'unités d'oxyde d'éthylène ou à partir d'unités d'oxyde d'éthylène et d'oxyde de propylène, avec une part maximale de 50 % en poids en unités d'oxyde de propylène, rapportée au poids de A,
K et K' représentent indépendamment l'un de l'autre une connectivité, sélectionnée à partir d'une liaison covalente ou à partir d'un fragment de molécule comportant deux valences libres,
T représente un fragment structurel, comprenant

- au moins un groupe $*\text{-}Si(OR)_x(R')_{3-x}$, dans lequel R et R' représentent indépendamment l'un de l'autre un groupe alkyle ($C_1$ à $C_4$) (en particulier du méthyle ou de l'éthyle), x représente 1, 2 ou 3, ou
- au moins un groupe anionique ou
- au moins un atome d'azote quaternaire,

Q représente un fragment structurel organique faisant office de centre, dérivé d'hydrocarbures linéaires, ramifiés, cycliques ou hétérocycliques, dans laquelle tous ceux-ci peuvent être respectivement saturés, insaturés ou aromatiques,
n représente un nombre entier allant de 3 à 64 (en particulier 3, 4, 5, 6, 7 ou 8).

4. Agent cosmétique de coloration selon une des revendications 2 ou 3, **caractérisé en ce que** le polymère en étoile faisant office de centre présente une particule de matière solide, laquelle est modifiée sur la surface avec au moins trois résidus *-K'-A-K-T, dans lesquels

A représente une chaîne polyoxyalkylène à partir d'unités d'oxyde d'éthylène ou à partir d'unités d'oxyde d'éthylène et d'oxyde de propylène, avec une part maximale de 50 % en poids en unités d'oxyde de propylène, rapportée au poids de A,
K et K' représentent indépendamment l'un de l'autre une connectivité, sélectionnée à partir d'une liaison covalente ou à partir d'un fragment de molécule comportant deux valences libres,
T représente un fragment structurel, comprenant

- au moins un groupe $*\text{-}Si(OR)_x(R')_{3-x}$, dans lequel R et R' représentent indépendamment l'un de l'autre un groupe alkyle ($C_1$ à $C_4$) (en particulier du méthyle ou de l'éthyle), x représente 1, 2 ou 3, ou
- au moins un groupe anionique ou
- au moins un atome d'azote quaternaire.

5. Agent cosmétique de coloration selon une des revendications 3 ou 4, **caractérisé en ce que** A représente un fragment structurel de la formule (A1)

$$*\text{-}(OCH_2CH_2)_n\text{-}(OCH_2CH(CH_3))_m\text{-}* \qquad (A1)$$

dans laquelle

n représente un nombre entier allant de 1 à 500,
m représente un nombre entier allant de 0 à 500 et
le fragment structurel de la formule (A1) possède une part maximale de 50 % en poids en unités d'oxyde de propylène, rapportée au poids du fragment structurel (A1).

6. Agent cosmétique de coloration selon une des revendications 3 à 5, **caractérisé en ce que** les résidus K et K' représentent indépendamment l'un de l'autre une liaison covalente, un groupe oxy, un groupe imino, un groupe alkényle en ($C_1$ à $C_6$) ou au moins une des connectivités (K1) à (K10) suivantes

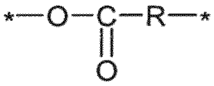
(K1)

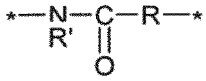
(K2)

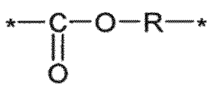
(K3)

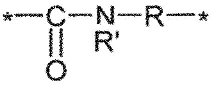
(K4)

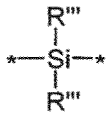
(K5)

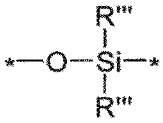
(K6)

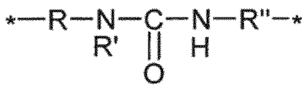
(K7)

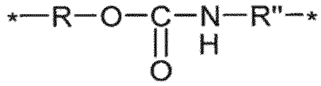
(K8)

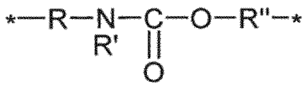
(K9)

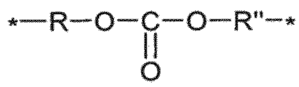
(K10)

dans lesquelles

R et R" représentent indépendamment l'un de l'autre du méthylène, de l'éthane-1,2-diyl, du propane-1,2-diyl, du propane-1,3-diyl, du butane-1,2-diyl, du butane-1,3-diyl, du butane-1,4-diyl ou du phénylène,
R' représente un atome d'hydrogène ou un groupe alkyle en ($C_1$ à $C_4$),
R"' représente indépendamment l'un de l'autre un groupe alkyle en ($C_1$ à $C_4$) ou un groupe aryle.

7. Agent cosmétique de coloration selon une des revendications 2 à 6, **caractérisé en ce qu'**il contient lesdits polymères en étoile dans une quantité allant de 0,01 à 10,0 % en poids, de manière préférée de 0,1 à 2,0 % en poids, de manière particulièrement préférée 0,2 à 1,0 % en poids, respectivement rapportée au poids de l'ensemble de l'agent.

**8.** Agent cosmétique de coloration selon une des revendications 2 à 7, **caractérisé en ce qu'**il contient en complément au moins un composé sélectionné parmi les amines organiques comprenant 2 à 20 atomes de carbone, les composés complexes de carboxylate du zinc, les composés d'alcoxydes du zinc, les composés complexes de carboxylate du plomb, les composés d'organoaluminium, les complexes métalliques de composés organiques de dicarbonyle, les complexes métalliques d'esters d'acide dicarboxylique organique.

**9.** Agent cosmétique de coloration selon une des revendications 2 à 8, **caractérisé en ce qu'**il contient en complément au moins un agent d'oxydation, en particulier du peroxyde d'hydrogène et/ou au moins un produit d'addition de celui-ci.

**10.** Procédé cosmétique de coloration de fibres kératiniques, lors duquel un agent cosmétique selon une des revendications 2 à 9 est appliqué sur les fibres kératiniques (en particulier des cheveux humains) puis est rincé après un temps d'action.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009024450 A1
- WO 2011015512 A1
- WO 2011015513 A1
- WO 2011015514 A1
- EP 2168633 A2
- EP 998908 A2